# EUROPEAN PATENT APPLICATION

(11) **EP 4 438 624 A1**
(43) Date of publication of application: **02.10.2024**
(21) Application number: 23164347.9
(22) Date of filing: 27.03.2023
(51) Int. Cl.: C07K 16/28, A61P 35/00

(54) **ANTIBODIES THAT BIND NECTIN-4 AND GAMMA-DELTA T CELL RECEPTORS**

(71) Applicant: LAVA Therapeutics N.V., 3584 CM Utrecht (NL)
(72) Inventor: VAN HELDEN, Paula Maria Wilhelmina, 3584 CM Utrecht (NL); ROOVERS, Robertus Cornelis, 3584 CM Utrecht (NL); LUTJE HULSIK, David, 3584 CM Utrecht (NL); VAN DER VLIET, Johannes Jelle, 3584 CM Utrecht (NL)
(74) Representative: Cooley (UK) LLP

(57) **Abstract**

The present invention relates to antibodies capable of binding human Nectin-4 and to multispecific antibodies capable of binding human Nectin-4 and capable of binding a human Vγ9Vδ2 T cell receptor. The invention further relates to pharmaceutical compositions comprising the antibodies of the invention and to uses of the antibodies of the invention for medical treatment.

## Description

### Field of the invention

The present invention relates to antibodies capable of binding human Nectin-4 and to multispecific antibodies capable of binding human Nectin-4 and capable of binding the Vδ2 chain or Vγ9 chain of a human Vγ9Vδ2 T cell receptor. The invention further relates to pharmaceutical compositions comprising the antibodies of the invention and to uses of the antibodies of the invention for medical treatment.

### Background of the invention

Nectin-4, also known as poliovirus receptor-like 4 (PVRL4), is a type I transmembrane 66 kDa polypeptide member of the Nectin family. In adults, human Nectin-4 is mainly expressed in the placenta with low expression in other tissues including the skin, bladder, salivary gland, oesophagus, breast, stomach, prostate, lung and trachea. Nectin-4 is involved in forming and maintaining adherens junctions together with cadherins and is a stimulatory co-receptor for the prolactin receptor (Heath and Rosenberg Nature Reviews Urology 18, 93-103 (2021).

Aberrant expression of Nectin-4 has been observed in several cancer types, including bladder, breast, lung, pancreatic and ovarian cancer. Nectin-4 has been associated with promoting cancer cell proliferation and metastasis via activation of WNT-β-catenin and Rac small G protein in the PI3K-AKT signaling pathway (Siddharth, S. et al. Int. J. Biochem. Cell Biol. 89, 85-94 (2017); Zhang et al. Hum. Pathol. 72: 107-116 (2018). Nectin-4 also interacts with the tyrosine kinase receptor ERBB2 to promote its activation, resulting in stimulation of the PI3K-AKT signalling pathway (Kedashiro et al. Sci. Rep. 9, 18997 (2019).

Nectin-4 targeting antibodies have been described. Enfortumab vedotin is an anti-Nectin-4 antibody linked to the cytotoxic agent monomethyl auristatin E approved for the treatment of urothelial cancer.

WO21257525 (BioAtla) describes CD3-binding T-cell engaging antibodies that target Nectin-4. Bispecific T-cell engaging antibodies have a tumor target binding specificity and a CD3-directed T-cell binding specificity and thus boost efficacy by re-directing T-cell cytotoxicity to malignant cells, see e.g. Huehls et al. (2015) Immunol Cell Biol 93:290; Ellerman (2019) Methods, 154:102. However, results vary significantly. For example, in one study in which a CD3 binding moiety was combined with binding moieties against 8 different B-cell targets (CD20, CD22, CD24, CD37, CD70, CD79b, CD138 and HLA-DR), it was found that the bispecific antibodies targeting the different tumor targets showed strong variation in their capacity to induce target cell cytotoxicity and that cytotoxicity did not correlate with antigen expression levels. For example, CD3-based bispecific antibodies targeting HLA-DR or CD138 were not able to induce cytotoxicity, in spite of intermediate to high HLA-DR and CD138 expression levels (Engelberts et al. (2020) Ebiomedicine 52:102625). Few T-cell redirecting therapies have reached late-stage clinical development, possibly due to significant toxicity, manufacturing problems, immunogenicity, narrow therapeutic windows and low response rates. In particular, toxicity may occur when the T-cell engager includes a CD3 binding arm and results in uncontrolled and exaggerated immune activation and cytokine release.

Thus, while significant progress has been made, there is still a need for novel Nectin-4 targeting antibodies, such as T-cell engaging Nectin-4 targeting antibodies, that are therapeutically effective against a range of (target) tumor cells yet have acceptable toxicity, as well as good stability, developability and manufacturability properties.

### Summary of the invention

The present invention provides novel antibodies for Nectin-4-based therapy, such as Nectin-4 based cancer therapy. Novel Nectin-4 binding antibodies were identified. Furthermore, bispecific antibodies were constructed in which single-domain Nectin-4-binding regions were combined with binding regions capable of binding the human Vγ9Vδ2 T cell receptor and thus capable of engaging γδ T cells.

Accordingly, in a first aspect, the invention provides a multispecific antibody comprising a first antigen-binding region capable of binding human Nectin-4 and a second antigen-binding region capable of binding a human Vγ9Vδ2 T cell receptor, wherein the multispecific antibody is capable of competing for binding to human Nectin-4 with an antibody having the sequence set forth in SEQ ID NO: 1.

In a further aspect, the invention provides a multispecific antibody comprising a first antigen-binding region capable of binding human Nectin-4 and a second antigen-binding region capable of binding a human Vγ9Vδ2 T cell receptor, wherein the multispecific antibody is capable of competing for binding to human Nectin-4 with an antibody having the sequence set forth in SEQ ID NO:9.

In a further aspect, the invention provides a multispecific antibody comprising a first antigen-binding region capable of binding human Nectin-4 and a second antigen-binding region capable of binding a human Vγ9Vδ2 T cell receptor, wherein the multispecific antibody is capable of competing for binding to human Nectin-4 with an antibody having the sequence set forth in SEQ ID NO: 17.

In a further aspect, the invention provides a multispecific antibody comprising a first antigen-binding region capable of binding human Nectin-4 and a second antigen-binding region capable of binding a human Vγ9Vδ2 T cell receptor, wherein the multispecific antibody is capable of competing for binding to human Nectin-4 with an antibody having the sequence set forth in SEQ ID NO:25.

In a further main aspect, the invention provides an antibody comprising a first antigen-binding region capable of binding human Nectin-4, wherein the antibody is capable of competing with the antibody set forth in SEQ ID NO: 1.

In a further aspect, the invention provides an antibody comprising a first antigen-binding region capable of binding human Nectin-4, wherein the antibody is capable of competing for binding to human Nectin-4 with an antibody having the sequence set forth in SEQ ID NO:9.

In a further aspect, the invention provides an antibody comprising a first antigen-binding region capable of binding human Nectin-4, wherein the antibody is capable of competing for binding to human Nectin-4 with an antibody having the sequence set forth in SEQ ID NO:17.

In a further aspect, the invention provides an antibody comprising a first antigen-binding region capable of binding human Nectin-4, wherein the antibody is capable of competing for binding to human Nectin-4 with an antibody having the sequence set forth in SEQ ID NO:25.

Further aspects and embodiments of the invention are described below.

### Brief description of the drawings

**Figure 1****:** Binding of VHHs to CHO-K1 cells expressing human Nectin-4.
**Figure 2****:** Binding of VHHs to CHO-K1 cells expressing cynomolgus monkey and murine Nectin-4.
**Figure 3****:** Binding of VHH to tumor cell lines.
**Figure 4****:** Binding of Nectin-4xVδ2 bispecific VHHs to tumor cell lines.
**Figure 5****:** Binding of Nectin-4xVδ2 bispecific VHHs to Vγ9Vδ2 T cells.
**Figure 6****:** Vγ9Vδ2-T cell degranulation and cytotoxicity of Nectin-4 expressing A-431 tumor cells induced by Nectin-4xVδ2 bispecific VHHs.

### Detailed description of the invention

### Definitions

The term "human Nectin-4", when used herein, refers to the human Nectin-4 protein (UniProt Q96NY8 · NECT4_HUMAN). The sequence of human Nectin-4 is set forth in SEQ ID NO:44.

The term "human Vδ2", when used herein, refers to the rearranged δ2 chain of the Vγ9Vδ2-T cell receptor (TCR) (SEQ ID NO:45). UniProtKB - A0JD36 (A0JD36_HUMAN) gives an example of a variable TRDV2 sequence.

The term "human Vy9", when used herein, refers to the rearranged y9 chain of the Vγ9Vδ2-T cell receptor (TCR). UniProtKB - Q99603_HUMAN gives an example of a variable TRGV9 sequence.

The term "antibody" is intended to refer to an immunoglobulin molecule, a fragment of an immunoglobulin molecule or a derivative of either thereof that has the ability to specifically bind to an antigen under typical physiological conditions with a half-life of significant periods of time, such as at least about 30 minutes, at least about one hour, at least about two hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to recruit an effector activity). Antigen-binding regions which interact with an antigen may comprise variable regions of both the heavy and light chains of an immunoglobulin molecule or may comprise or consist of single-domain antigen-binding regions, for example a heavy chain- or light chain variable region only. The constant regions of an antibody, if present, may mediate the binding of the immunoglobulin to host tissues or factors, including various cells of the immune system (such as effector cells and T cells) and components of the complement system such as C1q, the first component in the classical pathway of complement activation. In some embodiments, however, the Fc region of the antibody has been modified to become inert, "inert" means an Fc region which is at least not able to bind any Fc receptor, such as any Fcy receptor, induce Fc-mediated cross-linking of FcRs, or induce FcR-mediated cross-linking of target antigens via two Fc regions of individual antibodies. In a further embodiment, the inert Fc region is in addition not able to bind C1q. In one embodiment, the antibody contains mutations at positions 234 and 235 (Canfield and Morrison (1991) 3 Exp Med 173:1483), e.g. a Leu to Phe mutation at position 234 and a Leu to Glu mutation at position 235 (according to the EU-numbering, see below). In another embodiment, the antibody contains a Leu to Ala mutation at position 234, a Leu to Ala mutation at position 235 and a Pro to Gly mutation at position 329. In another embodiment, the antibody contains a Leu to Phe mutation at position 234, a Leu to Glu mutation at position 235 and an Asp to Ala at position 265.

The Fc region of an immunoglobulin is defined as the fragment of an antibody which would be typically generated after digestion of an antibody with papain which includes the two CH2-CH3 regions of an immunoglobulin and a connecting region, e.g. a hinge region. The constant domain of an antibody heavy chain defines the antibody isotype, e.g. IgG1, IgG2, IgG3, IgG4, IgA1, IgA2, IgM, IgD, or IgE. The Fc-region mediates the effector functions of antibodies with cell surface receptors called Fc receptors and proteins of the complement system.

The term "hinge region" as used herein is intended to refer to the hinge region of an immunoglobulin heavy chain. Thus, for example, the hinge region of a human IgG1 antibody corresponds to amino acids 216-230 according to the EU numbering.

The term "CH2 region" or "CH2 domain" as used herein is intended to refer to the CH2 region of an immunoglobulin heavy chain. Thus, for example the CH2 region of a human IgG1 antibody corresponds to amino acids 231-340 according to the EU numbering. However, the CH2 region may also be any of the other subtypes as described herein.

The term "CH3 region" or "CH3 domain" as used herein is intended to refer to the CH3 region of an immunoglobulin heavy chain. Thus, for example the CH3 region of a human IgG1 antibody corresponds to amino acids 341-447 according to the EU numbering. However, the CH3 region may also be any of the other subtypes as described herein.

Reference to amino acid positions in the Fc region/Fc domain in the present invention is according to the EU-numbering (Edelman et al., Proc Natl Acad Sci U S A. 1969 May;63(1):78-85; Kabat et al., Sequences of proteins of immunological interest. 5th Edition - 1991 NIH Publication No. 91-3242).

As indicated above, the term antibody as used herein, unless otherwise stated or clearly contradicted by context, includes fragments of an antibody that retain the ability to specifically bind to the antigen. It has been shown that the antigen-binding function of an antibody may be performed by fragments of a full-length antibody. Examples of binding fragments encompassed within the term "antibody" include (i) a Fab' or Fab fragment, i.e. a monovalent fragment consisting of the VL, VH, CL and CH1 domains, or a monovalent antibody as described in WO2007059782; (ii) F(ab')2 fragments, i.e. bivalent fragments comprising two Fab fragments linked by a disulfide bridge at the hinge region; (iii) a Fd fragment consisting essentially of the VH and CH1 domains; and (iv) a Fv fragment consisting essentially of the VL and VH domains of a single arm of an antibody. Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain antibodies or single chain Fv (scFv), see for instance Bird et al., Science 242, 423-426 (1988) and Huston et al., PNAS USA 85, 5879-5883 (1988)). Such single-chain antibodies are encompassed within the term antibody unless otherwise indicated by context. The term antibody, unless specified otherwise, also includes polyclonal antibodies, monoclonal antibodies (mAbs), chimeric antibodies and humanized antibodies, and antibody fragments provided by any known technique, such as enzymatic cleavage, peptide synthesis and recombinant techniques.

In some embodiments of the antibodies of the invention, the first antigen-binding region or the second antigen-binding region, or both, is a single-domain antibody. Single-domain antibodies (sdAb, also called Nanobody^{®}, or VHH) are well known to the skilled person, see e.g. Hamers-Casterman et al. (1993) Nature 363:446, Roovers et al. (2007) Curr Opin Mol Ther 9:327 and Krah et al. (2016) Immunopharmacol Immunotoxicol 38:21. Single-domain antibodies comprise a single CDR1, a single CDR2 and a single CDR3. Examples of single-domain antibodies are variable fragments of heavy-chain-only antibodies, antibodies that naturally do not comprise light chains, single-domain antibodies derived from conventional antibodies and engineered antibodies. Single-domain antibodies may be derived from any species. For example, naturally occurring VHH molecules can be derived from antibodies raised in Camelidae species, for example in camel, dromedary, llama, alpaca and guanaco. Like a whole antibody, a single-domain antibody is able to bind selectively to a specific antigen. Single-domain antibodies may contain only the variable domain of an immunoglobulin chain, i.e. CDR1, CDR2 and CDR3 and framework regions.

The term "immunoglobulin" as used herein is intended to refer to a class of structurally related glycoproteins typically consisting of two pairs of polypeptide chains, one pair of light (L) chains and one pair of heavy (H) chains, all four potentially inter-connected by disulfide bonds, although some mammalian species also produce heavy-chain only antibodies. The term "immunoglobulin heavy chain", "heavy chain of an immunoglobulin" or "heavy chain" as used herein is intended to refer to one of the chains of an immunoglobulin. A heavy chain is typically comprised of a heavy chain variable region (abbreviated herein as VH) and a heavy chain constant region (abbreviated herein as CH) which defines the isotype of the immunoglobulin. The heavy chain constant region typically is comprised of three domains, CH1, CH2, and CH3. The heavy chain constant region further comprises a hinge region. Within the structure of the immunoglobulin (e.g. IgG), the two heavy chains are inter-connected via disulfide bonds in the hinge region. Equally to the heavy chains, each light chain is typically comprised of several regions: a light chain variable region (VL) and a light chain constant region (CL). Furthermore, the VH and VL regions may be subdivided into regions of hypervariability (or hypervariable regions which may be hypervariable in sequence and/or form structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4. CDR sequences may be determined by use of various methods, e.g. the methods provided by Kabat et al. (1991) Sequence of protein of immunological interest, fifth edition. NIH publication or Choitia and Lesk (1987) J. Mol. Biol. 196:901. Various methods for CDR determination and amino acid numbering can be compared on www.abysis.org (UCL).

The term "isotype" as used herein, refers to the immunoglobulin (sub)class (for instance IgG1, IgG2, IgG3, IgG4, IgD, IgA, IgE, or IgM) or any allotype thereof, such as IgG1m(za) and IgG1m(f) that is encoded by heavy chain constant region genes. Each heavy chain isotype can be combined with either a kappa (κ) or lambda (λ) light chain. An antibody of the invention can possess any isotype.

The term "parent antibody", is to be understood as an antibody which is identical to an antibody according to the invention, but wherein the parent antibody does not have one or more of the specified mutations. A "variant" or "antibody variant" or a "variant of a parent antibody" of the present invention is an antibody molecule which comprises one or more mutations (substitutions, deletions or insertions) as compared to a "parent antibody". Amino acid substitutions may exchange a native amino acid for another naturally-occurring amino acid, or for a non-naturally-occurring amino acid derivative. The amino acid substitution may be conservative or non-conservative. In the context of the present invention, conservative substitutions may be defined by substitutions within the classes of amino acids reflected in one or more of the following three tables:

### Amino acid residue classes for conservative substitutions

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

### Alternative conservative amino acid residue substitution classes

| | | | |
|---|---|---|---|
| 1 | A | S | T |
| 2 | D | E | |
| 3 | N | Q | |
| 4 | R | K | |
| 5 | I | L | M |
| 6 | F | Y | W |

### Alternative Physical and Functional Classifications of Amino Acid Residues

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P, and T |
| Flexible residues | Q, T, K, S, G, N, D, E, and R |

In the context of the present invention, a substitution in a variant is indicated as:
Original amino acid - position - substituted amino acid;

The three-letter code, or one letter code, are used, including the codes Xaa and X to indicate amino acid residue. Accordingly, the notation "T366W" means that the variant comprises a substitution of threonine with tryptophan in the variant amino acid position corresponding to the amino acid in position 366 in the parent antibody.

Furthermore, the term "a substitution" includes a substitution into any one of the other nineteen natural amino acids, or into other amino acids, such as non-natural amino acids. For example, a substitution of amino acid T in position 366 includes each of the following substitutions: 366A, 366C, 366D, 366G, 366H, 366F, 366I, 366K, 366L, 366M, 366N, 366P, 366Q, 366R, 366S, 366E, 366V, 366W, and 366Y.

The term "full-length antibody" when used herein, refers to an antibody which contains all heavy and light chain constant and variable domains corresponding to those that are normally found in a wild-type antibody of that isotype.

The term "chimeric antibody" refers to an antibody wherein the variable region is derived from a non-human species (e.g. derived from rodents) and the constant region is derived from a different species, such as human. Chimeric antibodies may be generated by genetic engineering. Chimeric monoclonal antibodies for therapeutic applications are developed to reduce antibody immunogenicity.

The term "humanized antibody" refers to a genetically engineered non-human antibody, which contains human antibody constant domains and non-human variable domains modified to contain a high level of sequence homology to human variable domains. This can be achieved by grafting of the six non-human antibody complementarity-determining regions (CDRs), which together form the antigen binding site, onto a homologous human acceptor framework region (FR). In order to fully reconstitute the binding affinity and specificity of the parental antibody, the substitution of framework residues from the parental antibody (i.e. the non-human antibody) into the human framework regions (back-mutations) may be required. Structural homology modeling may help to identify the amino acid residues in the framework regions that are important for the binding properties of the antibody. Thus, a humanized antibody may comprise non-human CDR sequences, primarily human framework regions optionally comprising one or more amino acid back-mutations to the non-human amino acid sequence, and, optionally, fully human constant regions. Optionally, additional amino acid modifications, which are not necessarily back-mutations, may be introduced to obtain a humanized antibody with preferred characteristics, such as affinity and biochemical properties. Humanization of non-human therapeutic antibodies is performed to minimize its immunogenicity in man while such humanized antibodies at the same time maintain the specificity and similar binding affinity of the antibody of non-human origin.

The term "multispecific antibody" refers to an antibody having specificities for at least two different, such as at least three, typically non-overlapping, epitopes, due to the presence of two or more antigen-binding regions. Such epitopes may be on the same or on different target antigens. If the epitopes are on different targets, such targets may be on the same cell or different cells or cell types.

The term "bispecific antibody" refers to an antibody having specificities for two different, typically non-overlapping, epitopes, due to the presence of two antigen-binding regions. Such epitopes may be on the same or different targets. If the epitopes are on different targets, such targets may be on the same cell or different cells or cell types.

Examples of different classes of bispecific antibodies include but are not limited to (i) IgG-like molecules with complementary CH3 domains to force heterodimerization; (ii) recombinant IgG-like dual targeting molecules, wherein the two sides of the molecule each contain the Fab fragment or part of the Fab fragment of at least two different antibodies; (iii) IgG fusion molecules, wherein full length IgG antibodies are fused to extra Fab fragment or parts of Fab fragment; (iv) Fc fusion molecules, wherein single chain Fv molecules or stabilized diabodies are fused to heavy-chain constant- domains, Fc-regions or parts thereof; (v) Fab fusion molecules, wherein different Fab fragments are fused together, fused to heavy-chain constant-domains, Fc-regions or parts thereof; and (vi) scFv-and diabody-based and heavy chain-based antibodies (e.g., domain antibodies, Nanobodies^{®}) wherein different single chain Fv molecules or different diabodies or different heavy-chain antibodies (e.g. domain antibodies, Nanobodies^{®}) are fused to each other or to another protein or carrier molecule fused to heavy-chain constant-domains, Fc-regions or parts thereof.

Examples of IgG-like molecules with complementary CH3 domains molecules include but are not limited to the Triomab^{®} (Trion Pharma/Fresenius Biotech), the Knobs-into-Holes (Genentech), CrossMAbs (Roche) and the electrostatically-matched (Amgen, Chugai, Oncomed), the LUZ-Y (Genentech, Wranik et al. J. Biol. Chem. 2012, 287(52): 43331-9, doi: 10.1074/jbc.M112.397869. Epub 2012 Nov 1), DIG-body and PIG-body (Pharmabcine, WO2010134666, WO2014081202), the Strand Exchange Engineered Domain body (SEEDbody)(EMD Serono), the Biclonics (Merus, WO2013157953), FcΔAdp (Regeneron), bispecific IgG1 and IgG2 (Pfizer/Rinat), Azymetric scaffold (Zymeworks/Merck,), mAb-Fv (Xencor), bivalent bispecific antibodies (Roche, WO2009080254) and DuoBody^{®} molecules (Genmab).

Examples of recombinant IgG-like dual targeting molecules include, but are not limited to, Dual Targeting (DT)-Ig (GSK/Domantis, WO2009058383), Two-in-one Antibody (Genentech, Bostrom, et al 2009. Science 323, 1610-1614), Cross-linked Mabs (Karmanos Cancer Center), mAb2 (F-Star), Zybodies^{™} (Zyngenia, LaFleur et al. MAbs. 2013 Mar-Apr;5(2):208-18), approaches with common light chain, κλBodies (NovImmune, WO2012023053), US2020319181 (Merus) and CovX-body^{®} (CovX/Pfizer, Doppalapudi, V.R., et al 2007. Bioorg. Med. Chem. Lett. 17,501-506).

Examples of IgG fusion molecules include but are not limited to Dual Variable Domain (DVD)-Ig (Abbott), Dual domain double head antibodies (Unilever; Sanofi Aventis), IgG-like Bispecific (ImClone/Eli Lilly, Lewis et al. Nat Biotechnol. 2014 Feb;32(2):191-8), Ts2Ab (MedImmune/AZ, Dimasi et al. J Mol Biol. 2009 Oct 30;393(3):672-92) and BsAb (Zymogenetics, WO2010111625), HERCULES (Biogen Idec), scFv fusion (Novartis), scFv fusion (Changzhou Adam Biotech Inc) and TvAb (Roche).

Examples of Fc fusion molecules include but are not limited to scFv/Fc Fusions (Academic Institution, Pearce et al Biochem Mol Biol Int. 1997 Sep;42(6):1179), SCORPION (Emergent BioSolutions/Trubion, Blankenship JW, et al. AACR 100th Annual meeting 2009 (Abstract #5465); Zymogenetics/BMS, WO2010111625), Dual Affinity Retargeting Technology (Fc-DARTTM) (MacroGenics) and Dual(ScFv)2-Fab (National Research Center for Antibody Medicine - China).

Examples of Fab fusion bispecific antibodies include but are not limited to F(ab)2 (Medarex/AMGEN), Dual-Action or Bis-Fab (Genentech), Dock-and-Lock^{®} (DNL) (ImmunoMedics), Bivalent Bispecific (Biotecnol) and Fab-Fv (UCB-Celltech).

Examples of scFv-, diabody-based and domain antibodies include but are not limited to Bispecific T Cell Engager (BiTE^{®}) (Micromet, Tandem Diabody (Tandab) (Affimed), Dual Affinity Retargeting Technology (DARTTM) (MacroGenics), Single-chain Diabody (Academic, Lawrence FEBS Lett. 1998 Apr 3;425(3):479-84), TCR-like Antibodies (AIT, ReceptorLogics), Human Serum Albumin ScFv Fusion (Merrimack, WO2010059315) and COMBODY molecules (Epigen Biotech, Zhu et al. Immunol Cell Biol. 2010 Aug;88(6):667-75), dual targeting nanobodies^{®} (Ablynx, Hmila et al., FASEB J. 2010), dual targeting heavy chain only domain antibodies. In some embodiments, the multispecific antibody of the invention is in a VHH-Fc format, i.e. the antibody comprises two or more single-domain antigen-binding regions that are linked to each other via a human Fc region dimer. In this format, each single-domain antigen-binding region is fused to an Fc region polypeptide and the two fusion polypeptides form a dimeric bispecific antibody via disulfide bridges in the hinge region. Such constructs typically do not contain full, or any, CH1 or light chain sequences. Figure 12B of WO06064136 provides an illustration of an example of this embodiment.

In the context of antibody binding to an antigen, the terms "binds" or "specifically binds" refer to the binding of an antibody to a predetermined antigen or target (e.g. human Nectin-4 or Vδ2) to which binding typically is with an affinity corresponding to a K_{D} of about 10⁻⁶ M or less, e.g. 10⁻⁷ M or less, such as about 10⁻⁸ M or less, such as about 10⁻⁹ M or less, about 10⁻¹⁰ M or less, or about 10⁻¹¹ M or even less, e.g. when determined as described in the Examples herein. K_{D} values can for example be determined using for instance surface plasmon resonance (SPR) technology in a BIAcore T200 or bio-layer interferometry (BLI) in an Octet RED96 instrument using the antigen as the ligand and the binding moiety or binding molecule as the analyte. Specific binding means that the antibody binds to the predetermined antigen with an affinity corresponding to a K_{D} that is at least tenfold lower, such as at least 100-fold lower, for instance at least 1,000 fold lower, such as at least 10,000 fold lower, for instance at least 100,000 fold lower than its affinity for binding to a non-specific antigen (e.g., BSA, casein) other than the predetermined antigen or a closely-related antigen. The degree with which the affinity is lower is dependent on the K_{D} of the binding moiety or binding molecule, so that when the K_{D} of the binding moiety or binding molecule is very low (that is, the binding moiety or binding molecule is highly specific), then the degree with which the affinity for the antigen is lower than the affinity for a non-specific antigen may be at least 10,000-fold. The term "K_{D}" (M), as used herein, refers to the dissociation equilibrium constant of a particular interaction between the antigen and the binding moiety or binding molecule.

In the context of the present invention, "capable of competing" or "able to compete" or "competes" refers to any detectably significant reduction in the propensity for a particular binding molecule (e.g. a Nectin-4 antibody) to bind a particular binding partner (e.g. Nectin-4) in the presence of another molecule (e.g. a different Nectin-4 antibody) that binds the binding partner. Typically, competition means that saturating binding of the first antibody to the antigen (Nectin-4) blocks or prevents binding of the second antibody, as determined by, e.g., biolayer interferometry (BLI) as described in Example 6 herein using sufficient amounts of the two (or more) competing antibodies. Additional methods for determining binding specificity by competitive inhibition may be found in for instance Harlow et al., Antibodies: A Laboratory Manual, Cold Spring Harbor Laboratory Press, Cold Spring Harbor, N.Y., (1988), Colligan et al., eds., Current Protocols in Immunology, Greene Publishing Assoc, and Wiley InterScience N. Y., (1992, 1993), and Muller, Meth. Enzymol. 92, 589-601 (1983). In one embodiment, the antibody of the present invention binds to the same epitope on Nectin-4 as antibody LV1181, LV1184, LV1185 and/or LV1186 and/or to the same epitope on Vδ2 as antibody 5C8 or 6H4 (WO2015156673) or the variant of 5C8 set forth in SEQ ID NO:37. There are several methods available for mapping antibody epitopes on target antigens known in the art, including but not limited to: crosslinking coupled mass spectrometry, allowing identification of peptides that are part of the epitope, and X-ray crystallography identifying individual residues on the antigen that form the epitope. Epitope residues can be determined as being all amino acid residues with at least one atom less than or equal to 5 Å from the antibody. 5 Å was chosen as the epitope cutoff distance to allow for atoms within a van der Waals radius plus a possible water-mediated hydrogen bond. Next, epitope residues can be determined as being all amino acid residues with at least one atom less than or equal to 8 Å. Less than or equal to 8 Å is chosen as the epitope cutoff distance to allow for the length of an extended arginine amino acid. Crosslinking coupled mass spectrometry begins by binding the antibody and the antigen with a mass labeled chemical crosslinker. Next the presence of the complex is confirmed using high mass MALDI detection. Because after crosslinking chemistry the Ab/Ag complex is extremely stable, many various enzymes and digestion conditions can be applied to the complex to provide many different overlapping peptides. Identification of these peptides is performed using high resolution mass spectrometry and MS/MS techniques. Identification of the crosslinked peptides is determined using mass tag linked to the cross-linking reagents. After MS/MS fragmentation and data analysis, peptides that are crosslinked and are derived from the antigen are part of the epitope, while peptides derived from the antibody are part of the paratope. All residues between the most N- and C-terminal crosslinked residue from the individual crosslinked peptides found are considered to be part of the epitope or paratope.

The terms "first" and "second" antigen-binding regions when used herein do not refer to their orientation / position in the antibody, i.e. they have no meaning with regard to the N- or C-terminus. The terms "first" and "second" only serve to correctly and consistently refer to the two different antigen-binding regions in the claims and the description.

"Capable of binding the Vδ2 chain of a Vγ9Vδ2-TCR" means that the antibody can bind the Vδ2 chain as a separate molecule and/or as part of a Vγ9Vδ2-TCR. However, the antibody will not bind to the Vγ9 chain as a separate molecule.

"Capable of binding the Vγ9 chain of a Vγ9Vδ2-TCR" means that the antibody can bind the Vγ9 chain as a separate molecule and/or as part of a Vγ9Vδ2-TCR. However, the antibody will not bind to the Vδ2 chain as a separate molecule.

With "able to activate Vγ9Vδ2-T cells" in the context of the present invention is meant that Vγ9Vδ2-T cells are activated in the presence of a multispecific antibody, such as a bispecific antibody, of the invention in the presence of a target cell expressing Nectin-4. Preferably the activation of the Vγ9Vδ2-T cells is measurable through gene-expression and/or (surface) marker expression (e.g., activation markers, such as CD25, CD69, or CD107a) and/or secretory protein (e.g., cytokines or chemokines) profiles. In a preferred embodiment, the antibody is able to induce activation (e.g. upregulation of CD69 and/or CD25 expression) resulting in degranulation (marked by an increase in CD107a expression; see e.g. Example 3 of WO2020060405) and cytokine production (e.g. TNFo, IFN-γ) by Vγ9Vδ2-T cells. Preferably, the antibody that is used is able to increase CD107a expression on Vγ9Vδ2-T cells to at least 10%, more preferably at least 20%, more preferably at least 40%, most preferably at least 90%, when used in an assay as described in Example 3 of WO2020060405, wherein e.g. 10% means that 10% of the total number of cells is positive for CD107a. In another embodiment, the number of cells positive for CD107a is increased 1.5-fold, such as 2-fold, e.g. 5-fold, in the presence of an antibody of the invention.

"% sequence identity", when used herein, refers to the number of identical nucleotide or amino acid positions shared by different sequences (i.e., % identity = # of identical positions/total # of positions x 100), taking into account the number of gaps, and the length of each gap, which need to be introduced for optimal alignment. The percent identity between two nucleotide or amino acid sequences may e.g. be determined using the algorithm of E. Meyers and W. Miller, Comput. Appl. Biosci 4, 11-17 (1988) which has been incorporated into the ALIGN program (version 2.0), using a PAM120 weight residue table, a gap length penalty of 12 and a gap penalty of 4.

### Further aspects and embodiments of the invention

As described above, in a first main aspect, the invention relates to a multispecific antibody comprising a first antigen-binding region capable of binding human Nectin-4 and a second antigen-binding region capable of binding a human Vγ9Vδ2-T cell receptor, wherein the multispecific antibody is capable of competing for binding to human Nectin-4 with an antibody having the sequence set forth in SEQ ID NO:1.

In a further aspect, the invention provides a multispecific antibody comprising a first antigen-binding region capable of binding human Nectin-4 and a second antigen-binding region capable of binding a human Vγ9Vδ2 T cell receptor, wherein the multispecific antibody is capable of competing for binding to human Nectin-4 with an antibody having the sequence set forth in SEQ ID NO:9.

In a further aspect, the invention provides a multispecific antibody comprising a first antigen-binding region capable of binding human Nectin-4 and a second antigen-binding region capable of binding a human Vγ9Vδ2 T cell receptor, wherein the multispecific antibody is capable of competing for binding to human Nectin-4 with an antibody having the sequence set forth in SEQ ID NO:17.

In a further aspect, the invention provides a multispecific antibody comprising a first antigen-binding region capable of binding human Nectin-4 and a second antigen-binding region capable of binding a human Vγ9Vδ2 T cell receptor, wherein the multispecific antibody is capable of competing for binding to human Nectin-4 with an antibody having the sequence set forth in SEQ ID NO:25.

In one embodiment, the multispecific antibody is a bispecific antibody. In another embodiment, the multispecific antibody is a trispecific antibody.

In one embodiment, the first antigen-binding region is a single-domain antibody, for example a single-domain antibody which consists of a heavy chain variable region. In one embodiment, the first antigen-binding region is humanized.

In one embodiment, the multispecific antibody is a bispecific antibody and the first antigen-binding region is a single-domain antibody.

In one embodiment, the multispecific antibody binds to the same epitope on Nectin-4 as an antibody having the sequence set forth in SEQ ID NO:1.

In one embodiment, the multispecific antibody is capable of competing for binding to human Nectin-4 with an antibody having the sequence set forth in SEQ ID NO:9.

In one embodiment, the multispecific antibody binds to the same epitope on Nectin-4 as an antibody having the sequence set forth in SEQ ID NO:9.

In one embodiment, the multispecific antibody is capable of competing for binding to human Nectin-4 with an antibody having the sequence set forth in SEQ ID NO:17.

In one embodiment, the multispecific antibody binds to the same epitope on Nectin-4 as an antibody having the sequence set forth in SEQ ID NO:17.

In one embodiment, the multispecific antibody is capable of competing for binding to human Nectin-4 with an antibody having the sequence set forth in SEQ ID NO:25.

In one embodiment, the multispecific antibody binds to the same epitope on Nectin-4 as an antibody having the sequence set forth in SEQ ID NO:25.

In one embodiment, the multispecific antibody is capable of competing for binding to human Nectin-4 with an antibody having the sequence set forth in SEQ ID NO:1, with an antibody having the sequence set forth in SEQ ID NO:9, with an antibody having the sequence set forth in SEQ ID NO:17 and with an antibody having the sequence set forth in SEQ ID NO:25.

In one embodiment of the multispecific antibody of the invention, the first antigen-binding region comprises:
- the CDR1 sequence set forth in SEQ ID NO:2, the CDR2 sequence set forth in SEQ ID NO:3 and the CDR3 sequence set forth in SEQ ID NO:4, or
- a CDR1 sequence which differs from the CDR1 sequence set forth in SEQ ID NO:2 in one or two positions, a CDR2 sequence which differs from the CDR2 sequence set forth in SEQ ID NO:3 in one, two, three, four or five positions and a CDR3 sequence which differs from the CDR3 sequence set forth in SEQ ID NO:4 in one or two positions,
wherein preferably the first antigen-binding region comprises or consists of:
- the sequence set forth in SEQ ID NO:1, SEQ ID NO:56, SEQ ID NO:57 or SEQ ID NO:58, or
- a sequence having at least 90%, such as least 92%, e.g. at least 94%, such as at least 96%, e.g. at least 98% sequence identity to the sequence ser forth in SEQ ID NO:1.

Thus, in one embodiment, the first antigen-binding region is a variant of antibody LV1184 described herein.

In one embodiment, the CDR1 sequence comprises or consists of five amino acids, wherein position 1 is I or L, position 2 is N, position 3 is L or I, position 4 is M and position G. In one embodiment, the CDR1 sequence comprises or consists of the sequence set forth in SEQ ID NO:2 or the sequence set forth in SEQ ID NO:18.

In one embodiment, the CDR2 sequence comprises or consists of sixteen amino acids, wherein position 1 is T or S, position 2 is I, position 3 is T or S, position 4 is R, P, T or S, position 5 is G, position 6 is G, position 7 is S, position 8 is T or V, position 9 is N or R, position 10 is Y, position 11 is A, position 12 is D, position 13 is S, position 14 is V, position 15 is K and position 16 is G.

In one embodiment, the CDR2 sequence comprises or consists of the sequence set forth in SEQ ID NO:3 or the sequence set forth in SEQ ID NO:11 or the sequence set forth in SEQ ID NO:19 or the sequence set forth in SEQ ID NO:27.

In one embodiment, the first antigen-binding region comprises one or more or all of the sequences set forth in SEQ ID NO:5, 6, 7 and 8.

In one embodiment, the first antigen-binding region comprises or consists of: the sequence set forth in SEQ ID NO: 1, or a sequence having at least 90%, such as least 92%, e.g. at least 94%, such as at least 96%, e.g. at least 98% sequence identity to the sequence set forth in SEQ ID NO: 1.

In one embodiment, the first antigen-binding region comprises or consists of the sequence set forth in SEQ ID NO:1.

In one embodiment of the multispecific antibody of the invention, the first antigen-binding region comprises the CDR1 sequence set forth in SEQ ID NO:2, the CDR2 sequence set forth in SEQ ID NO:3 and the CDR3 sequence set forth in SEQ ID NO:4 and comprises or consists of a sequence having at least 90%, such as least 92%, e.g. at least 94%, such as at least 96%, e.g. at least 98% sequence identity to the sequence ser forth in SEQ ID NO:1.

In one embodiment of the multispecific antibody of the invention, the first antigen-binding region comprises:
- the CDR1 sequence set forth in SEQ ID NO:10, the CDR2 sequence set forth in SEQ ID NO:11 and the CDR3 sequence set forth in SEQ ID NO:12, or
- a CDR1 sequence which differs from the CDR1 sequence set forth in SEQ ID NO:10 in one or two positions, a CDR2 sequence which differs from the CDR2 sequence set forth in SEQ ID NO:11 in one, two, three, four or five positions and a CDR3 sequence which differs from the CDR3 sequence set forth in SEQ ID NO:12 in one or two positions,
wherein preferably the first antigen-binding region comprises or consists of:
- the sequence set forth in SEQ ID NO:9, SEQ ID NO:53, SEQ ID NO:54 or SEQ ID NO:55, or
- a sequence having at least 90%, such as least 92%, e.g. at least 94%, such as at least 96%, e.g. at least 98% sequence identity to the sequence ser forth in SEQ ID NO:9.

Thus, in one embodiment, the first antigen-binding region is a variant of antibody LV1181 described herein.

In one embodiment, the CDR1 sequence comprises or consists of five amino acids, wherein position 1 is I or L, position 2 is N, position 3 is L or I, position 4 is M and position G. In one embodiment, the CDR1 sequence comprises or consists of the sequence set forth in SEQ ID NO:2 or the sequence set forth in SEQ ID NO:18.

In one embodiment, the CDR2 sequence comprises or consists of sixteen amino acids, wherein position 1 is T or S, position 2 is I, position 3 is T or S, position 4 is R, P, T or S, position 5 is G, position 6 is G, position 7 is S, position 8 is T or V, position 9 is N or R, position 10 is Y, position 11 is A, position 12 is D, position 13 is S, position 14 is V, position 15 is K and position 16 is G.

In one embodiment, the CDR2 sequence comprises or consists of the sequence set forth in SEQ ID NO:3 or the sequence set forth in SEQ ID NO:11 or the sequence set forth in SEQ ID NO:19 or the sequence set forth in SEQ ID NO:27.

In one embodiment, the first antigen-binding region comprises one or more or all of the sequences set forth in SEQ ID NO:13, 14, 15 and 16.

In one embodiment, the first antigen-binding region comprises or consists of: the sequence set forth in SEQ ID NO:9, or a sequence having at least 90%, such as least 92%, e.g. at least 94%, such as at least 96%, e.g. at least 98% sequence identity to the sequence set forth in SEQ ID NO:9.

In one embodiment, the first antigen-binding region comprises or consists of the sequence set forth in SEQ ID NO:9.

In one embodiment of the multispecific antibody of the invention, the first antigen-binding region comprises the CDR1 sequence set forth in SEQ ID NO:10, the CDR2 sequence set forth in SEQ ID NO:11 and the CDR3 sequence set forth in SEQ ID NO:12 and comprises or consists of a sequence having at least 90%, such as least 92%, e.g. at least 94%, such as at least 96%, e.g. at least 98% sequence identity to the sequence ser forth in SEQ ID NO:9.

In one embodiment of the multispecific antibody of the invention, the first antigen-binding region comprises:
- the CDR1 sequence set forth in SEQ ID NO:18, the CDR2 sequence set forth in SEQ ID NO:19 and the CDR3 sequence set forth in SEQ ID NO:20, or
- a CDR1 sequence which differs from the CDR1 sequence set forth in SEQ ID NO:18 in one or two positions, a CDR2 sequence which differs from the CDR2 sequence set forth in SEQ ID NO:19 in one, two, three, four or five positions and a CDR3 sequence which differs from the CDR3 sequence set forth in SEQ ID NO:20 in one or two positions,
wherein preferably the first antigen-binding region comprises or consists of:
- the sequence set forth in SEQ ID NO: 17, SEQ ID NO:59, SEQ ID NO:60 or SEQ ID NO:61, or
- a sequence having at least 90%, such as least 92%, e.g. at least 94%, such as at least 96%, e.g. at least 98% sequence identity to the sequence ser forth in SEQ ID NO:17.

Thus, in one embodiment, the first antigen-binding region is a variant of antibody LV1185 described herein.

In one embodiment, the CDR1 sequence comprises or consists of five amino acids, wherein position 1 is I or L, position 2 is N, position 3 is L or I, position 4 is M and position G. In one embodiment, the CDR1 sequence comprises or consists of the sequence set forth in SEQ ID NO:2 or the sequence set forth in SEQ ID NO:18.

In one embodiment, the CDR2 sequence comprises or consists of sixteen amino acids, wherein position 1 is T or S, position 2 is I, position 3 is T or S, position 4 is R, P, T or S, position 5 is G, position 6 is G, position 7 is S, position 8 is T or V, position 9 is N or R, position 10 is Y, position 11 is A, position 12 is D, position 13 is S, position 14 is V, position 15 is K and position 16 is G.

In one embodiment, the CDR2 sequence comprises or consists of the sequence set forth in SEQ ID NO:3 or the sequence set forth in SEQ ID NO:11 or the sequence set forth in SEQ ID NO:19 or the sequence set forth in SEQ ID NO:27.

In one embodiment, the first antigen-binding region comprises one or more or all of the sequences set forth in SEQ ID NO:21, 22, 23 and 24.

In one embodiment, the first antigen-binding region comprises or consists of: the sequence set forth in SEQ ID NO:17, or a sequence having at least 90%, such as least 92%, e.g. at least 94%, such as at least 96%, e.g. at least 98% sequence identity to the sequence set forth in SEQ ID NO: 17.

In one embodiment, the first antigen-binding region comprises or consists of the sequence set forth in SEQ ID NO:17.

In one embodiment of the multispecific antibody of the invention, the first antigen-binding region comprises the CDR1 sequence set forth in SEQ ID NO:18, the CDR2 sequence set forth in SEQ ID NO:19 and the CDR3 sequence set forth in SEQ ID NO:20 and comprises or consists of a sequence having at least 90%, such as least 92%, e.g. at least 94%, such as at least 96%, e.g. at least 98% sequence identity to the sequence ser forth in SEQ ID NO:17.

In one embodiment of the multispecific antibody of the invention, the first antigen-binding region comprises:
- the CDR1 sequence set forth in SEQ ID NO:26, the CDR2 sequence set forth in SEQ ID NO:27 and the CDR3 sequence set forth in SEQ ID NO:28, or
- a CDR1 sequence which differs from the CDR1 sequence set forth in SEQ ID NO:26 in one or two positions, a CDR2 sequence which differs from the CDR2 sequence set forth in SEQ ID NO:27 in one, two, three, four or five positions and a CDR3 sequence which differs from the CDR3 sequence set forth in SEQ ID NO:28 in one or two positions,
wherein preferably the first antigen-binding region comprises or consists of:
- the sequence set forth in SEQ ID NO:25, SEQ ID NO:62, SEQ ID NO:63 or SEQ ID NO:64, or
- a sequence having at least 90%, such as least 92%, e.g. at least 94%, such as at least 96%, e.g. at least 98% sequence identity to the sequence ser forth in SEQ ID NO:25.

Thus, in one embodiment, the first antigen-binding region is a variant of antibody LV1186 described herein.

In one embodiment, the CDR1 sequence comprises or consists of five amino acids, wherein position 1 is I or L, position 2 is N, position 3 is L or I, position 4 is M and position G. In one embodiment, the CDR1 sequence comprises or consists of the sequence set forth in SEQ ID NO:2 or the sequence set forth in SEQ ID NO:18.

In one embodiment, the CDR2 sequence comprises or consists of sixteen amino acids, wherein position 1 is T or S, position 2 is I, position 3 is T or S, position 4 is R, P, T or S, position 5 is G, position 6 is G, position 7 is S, position 8 is T or V, position 9 is N or R, position 10 is Y, position 11 is A, position 12 is D, position 13 is S, position 14 is V, position 15 is K and position 16 is G.

In one embodiment, the CDR2 sequence comprises or consists of the sequence set forth in SEQ ID NO:3 or the sequence set forth in SEQ ID NO:11 or the sequence set forth in SEQ ID NO:19 or the sequence set forth in SEQ ID NO:27.

In one embodiment, the first antigen-binding region comprises one or more or all of the sequences set forth in SEQ ID NO:29, 30, 31 and 32.

In one embodiment, the first antigen-binding region comprises or consists of: the sequence set forth in SEQ ID NO:25, or a sequence having at least 90%, such as least 92%, e.g. at least 94%, such as at least 96%, e.g. at least 98% sequence identity to the sequence set forth in SEQ ID NO:25.

In one embodiment, the first antigen-binding region comprises or consists of the sequence set forth in SEQ ID NO:25.

In one embodiment of the multispecific antibody of the invention, the first antigen-binding region comprises the CDR1 sequence set forth in SEQ ID NO:26, the CDR2 sequence set forth in SEQ ID NO:27 and the CDR3 sequence set forth in SEQ ID NO:28 and comprises or consists of a sequence having at least 90%, such as least 92%, e.g. at least 94%, such as at least 96%, e.g. at least 98% sequence identity to the sequence ser forth in SEQ ID NO:25.

In one embodiment of the multispecific antibody of the invention, the first antigen-binding region comprises:
- the CDR1 sequence set forth in SEQ ID NO:66, the CDR2 sequence set forth in SEQ ID NO:67 and the CDR3 sequence set forth in SEQ ID NO:68, or
- a CDR1 sequence which differs from the CDR1 sequence set forth in SEQ ID NO:66 in one or two positions, a CDR2 sequence which differs from the CDR2 sequence set forth in SEQ ID NO:67 in one, two, three, four or five positions and a CDR3 sequence which differs from the CDR3 sequence set forth in SEQ ID NO:68 in one or two positions,
wherein preferably the first antigen-binding region comprises or consists of:
- the sequence set forth in SEQ ID NO:65, or
- a sequence having at least 90%, such as least 92%, e.g. at least 94%, such as at least 96%, e.g. at least 98% sequence identity to the sequence ser forth in SEQ ID NO:65.

In one embodiment of the multispecific antibody of the invention, the first antigen-binding region comprises:
- the CDR1 sequence set forth in SEQ ID NO:70, the CDR2 sequence set forth in SEQ ID NO:71 and the CDR3 sequence set forth in SEQ ID NO:72, or
- a CDR1 sequence which differs from the CDR1 sequence set forth in SEQ ID NO:70 in one or two positions, a CDR2 sequence which differs from the CDR2 sequence set forth in SEQ ID NO:71 in one, two, three, four or five positions and a CDR3 sequence which differs from the CDR3 sequence set forth in SEQ ID NO:72 in one or two positions,
wherein preferably the first antigen-binding region comprises or consists of:
- the sequence set forth in SEQ ID NO:69, or
- a sequence having at least 90%, such as least 92%, e.g. at least 94%, such as at least 96%, e.g. at least 98% sequence identity to the sequence ser forth in SEQ ID NO:69.

In one embodiment of the multispecific antibody of the invention, the first antigen-binding region comprises:
- the CDR1 sequence set forth in SEQ ID NO:74, the CDR2 sequence set forth in SEQ ID NO:75 and the CDR3 sequence set forth in SEQ ID NO:76, or
- a CDR1 sequence which differs from the CDR1 sequence set forth in SEQ ID NO:74 in one or two positions, a CDR2 sequence which differs from the CDR2 sequence set forth in SEQ ID NO:75 in one, two, three, four or five positions and a CDR3 sequence which differs from the CDR3 sequence set forth in SEQ ID NO:76 in one or two positions,
wherein preferably the first antigen-binding region comprises or consists of:
- the sequence set forth in SEQ ID NO:73, or
- a sequence having at least 90%, such as least 92%, e.g. at least 94%, such as at least 96%, e.g. at least 98% sequence identity to the sequence ser forth in SEQ ID NO:73.

In a further embodiment, the multispecific antibody of the invention has three antigen-binding regions wherein two of the antigen-binding regions bind Nectin-4. In such an embodiment, the two Nectin-4 binding regions may bind different epitopes or the same epitope on Nectin-4. The two Nectin-4 binding regions may be identical or have a different sequence.

As described above, the multispecific antibody of the invention comprises a second antigen-binding region capable of binding a human Vγ9Vδ2 T cell receptor.

In one embodiment, the second antigen-binding region is capable of binding the Vδ2 chain of a human Vγ9Vδ2 T cell receptor.

In another embodiment, the second antigen-binding region is capable of binding the Vγ9 chain of a human Vγ9Vδ2 T cell receptor.

Several antibodies which bind to Vδ2 or Vγ9 have been described in WO2015156673 and their antigen-binding regions or at least the CDR sequences thereof can be incorporated in the multispecific antibody of the invention.

In one embodiment, the second antigen-binding region is a single-domain antibody, for example a single-domain antibody which consists of a heavy chain variable region. In one embodiment, the second antigen-binding region is humanized.

In one embodiment, the multispecific antibody is a bispecific antibody, wherein the first antigen-binding region is a single-domain antibody and the second antigen-binding region is a single-domain antibody. Said bispecific antibody may optionally comprise further sequences, such as a linker and/or an immunoglobulin Fc region.

In one embodiment, the multispecific antibody is able to activate human Vγ9Vδ2 T cells. The activation of the Vγ9Vδ2 T cells may be measured through gene-expression and/or (surface) marker expression (e.g., activation markers, such as CD25, CD69, or CD107a) and/or secretory protein (e.g., cytokines or chemokines) profiles. In a preferred embodiment, the multispecific antibody is able to induce activation (e.g. upregulation of CD69 and/or CD25 expression) resulting in degranulation marked by an increase in CD107a expression (see the Examples herein) and/or cytokine production (e.g. TNFo, IFNγ) by Vγ9Vδ2 T cells. Preferably, a multispecific antibody of the present invention is able to increase the number of cells positive for CD107a at least 2-fold, such as at least 5-fold, when tested as described in Example 10 herein. In one embodiment, the multispecific antibody of the invention has an EC50 value for increasing the percentage of CD107a positive cells of 100 pM or less, such as 25 pM or less, e.g. 10 pM or less, when tested using Vγ9Vδ2 T cells and Nectin-4 expressing A-431 target cells as described herein in Example 10.

In one embodiment, the second antigen-binding region is capable of binding the Vδ2 chain of a human Vγ9Vδ2 T cell receptor and the multispecific antibody is capable of competing for binding to human Vδ2 with an antibody having the sequence set forth in SEQ ID NO:37.

In one embodiment, the second antigen-binding region is capable of binding the Vδ2 chain of a human Vγ9Vδ2 T cell receptor and the multispecific antibody is capable of competing for binding to human Vδ2 with an antibody having the sequence set forth in SEQ ID NO:38.

In one embodiment, the second antigen-binding region is capable of binding the Vδ2 chain of a human Vγ9Vδ2 T cell receptor and the multispecific antibody is capable of competing for binding to human Vδ2 with an antibody having the sequence set forth in SEQ ID NO:37 and is capable of competing for binding to human Vδ2 with an antibody having the sequence set forth in SEQ ID NO:38.

In a further embodiment, the multispecific antibody binds the same epitope on human Vδ2 as an antibody having the sequence set forth in SEQ ID NO:37.

In one embodiment, the multispecific antibody binds the same epitope on human Vδ2 as an antibody having the sequence set forth in SEQ ID NO:38.

In one embodiment of the multispecific antibody of the invention, the second antigen-binding region comprises:
(i) the VH CDR1 sequence set forth in SEQ ID NO:34, the VH CDR2 sequence set forth in SEQ ID NO:35 and the VH CDR3 sequence set forth in SEQ ID NO:36, wherein preferably X₁ is S, X₂ is F and X₃ is A, and wherein most preferably the second antigen-binding region comprises or consists of the sequence set forth in SEQ ID NO:37, or a sequence having at least 90%, such as least 92%, e.g. at least 94%, such as at least 96%, e.g. at least 98% sequence identity to the sequence set forth in SEQ ID NO:37, or
(ii) the VH CDR1 sequence set forth in SEQ ID NO:39, the VH CDR2 sequence set forth in SEQ ID NO:40 and the VH CDR3 sequence set forth in SEQ ID NO:41, wherein preferably the second antigen-binding region comprises or consists of the sequence set forth in SEQ ID NO:38, or a sequence having at least 90%, such as least 92%, e.g. at least 94%, such as at least 96%, e.g. at least 98% sequence identity to the sequence set forth in SEQ ID NO:38.

In one embodiment of the multispecific antibody of the invention, the second antigen-binding region comprises the VH CDR1 sequence set forth in SEQ ID NO:34, the VH CDR2 sequence set forth in SEQ ID NO:35 and the VH CDR3 sequence set forth in SEQ ID NO:36.

In one embodiment, X₁ in SEQ ID NO:34 is S.

In another embodiment, X₁ in SEQ ID NO:34 is G.

In one embodiment, X₂ in SEQ ID NO:36 is Y.

In another embodiment, X₂ in SEQ ID NO:36 is F.

In another embodiment, X₂ in SEQ ID NO:36 is S.

In one embodiment, X₃ in SEQ ID NO:36 is A.

In another embodiment, X₃ in SEQ ID NO:36 is K.

In one embodiment, X₁ in SEQ ID NO:34 is S and X₂ in SEQ ID NO:36 is Y.

In one embodiment, X₁ in SEQ ID NO:34 is S and X₂ in SEQ ID NO:36 is F.

In one embodiment, X₁ in SEQ ID NO:34 is S and X₂ in SEQ ID NO:36 is S.

In one embodiment, X₁ in SEQ ID NO:34 is G and X₂ in SEQ ID NO:36 is Y.

In one embodiment, X₁ in SEQ ID NO:34 is G and X₂ in SEQ ID NO:36 is F.

In one embodiment, X₁ in SEQ ID NO:34 is G and X₂ in SEQ ID NO:36 is S.

In one embodiment, X₁ in SEQ ID NO:34 is S and X₃ in SEQ ID NO:36 is A.

In one embodiment, X₁ in SEQ ID NO:34 is S and X₃ in SEQ ID NO:36 is K.

In one embodiment, X₁ in SEQ ID NO:34 is G and X₃ in SEQ ID NO:36 is A.

In one embodiment, X₁ in SEQ ID NO:34 is G and X₃ in SEQ ID NO:36 is K.

In one embodiment, X₂ in SEQ ID NO:34 is Y and X₃ in SEQ ID NO:36 is A.

In one embodiment, X₂ in SEQ ID NO:34 is Y and X₃ in SEQ ID NO:36 is K.

In one embodiment, X₂ in SEQ ID NO:34 is F and X₃ in SEQ ID NO:36 is A.

In one embodiment, X₂ in SEQ ID NO:34 is F and X₃ in SEQ ID NO:36 is K.

In one embodiment, X₂ in SEQ ID NO:34 is S and X₃ in SEQ ID NO:36 is A.

In one embodiment, X₂ in SEQ ID NO:34 is S and X₃ in SEQ ID NO:36 is K.

In one embodiment, X₁ in SEQ ID NO:34 is S, X₂ in SEQ ID NO:36 is Y and X₃ in SEQ ID NO:36 is A.

In one embodiment, X₁ in SEQ ID NO:34 is S, X₂ in SEQ ID NO:36 is F and X₃ in SEQ ID NO:36 is A.

In one embodiment, X₁ in SEQ ID NO:34 is S, X₂ in SEQ ID NO:36 is S and X₃ in SEQ ID NO:36 is A.

In one embodiment, X₁ in SEQ ID NO:34 is G, X₂ in SEQ ID NO:36 is Y and X₃ in SEQ ID NO:36 is A.

In one embodiment, X₁ in SEQ ID NO:34 is G, X₂ in SEQ ID NO:36 is F and X₃ in SEQ ID NO:36 is A.

In one embodiment, X₁ in SEQ ID NO:34 is G, X₂ in SEQ ID NO:36 is S and X₃ in SEQ ID NO:36 is A.

In one embodiment, X₁ in SEQ ID NO:34 is S, X₂ in SEQ ID NO:36 is Y and X₃ in SEQ ID NO:36 is K.

In one embodiment, X₁ in SEQ ID NO:34 is S, X₂ in SEQ ID NO:36 is F and X₃ in SEQ ID NO:36 is K.

In one embodiment, X₁ in SEQ ID NO:34 is S, X₂ in SEQ ID NO:36 is S and X₃ in SEQ ID NO:36 is K.

In one embodiment, X₁ in SEQ ID NO:34 is G, X₂ in SEQ ID NO:36 is Y and X₃ in SEQ ID NO:36 is K.

In one embodiment, X₁ in SEQ ID NO:34 is G, X₂ in SEQ ID NO:36 is F and X₃ in SEQ ID NO:36 is K.

In one embodiment, X₁ in SEQ ID NO:34 is G, X₂ in SEQ ID NO:36 is S and X₃ in SEQ ID NO:36 is K.

In one embodiment of the multispecific antibody of the invention, the second antigen-binding region comprises or consists of the sequence set forth in SEQ ID NO:37, or a sequence having at least 90%, such as least 92%, e.g. at least 94%, such as at least 96%, e.g. at least 98% sequence identity to the sequence set forth in SEQ ID NO:37.

In one embodiment of the multispecific antibody of the invention, the second antigen-binding region comprises or consists of the sequence set forth in SEQ ID NO:38, or a sequence having at least 90%, such as least 92%, e.g. at least 94%, such as at least 96%, e.g. at least 98% sequence identity to the sequence set forth in SEQ ID NO:38.

In one embodiment of the multispecific antibody of the invention,
(i) the first antigen-binding region comprises the VH CDR1 sequence set forth in SEQ ID NO:2, the VH CDR2 sequence set forth in SEQ ID NO:3 and the VH CDR3 sequence set forth in SEQ ID NO:4 and the second antigen-binding region comprises the VH CDR1 sequence set forth in SEQ ID NO:34, the VH CDR2 sequence set forth in SEQ ID NO:35 and the VH CDR3 sequence set forth in SEQ ID NO:36, or
(ii) the first antigen-binding region comprises the VH CDR1 sequence set forth in SEQ ID NO:10, the VH CDR2 sequence set forth in SEQ ID NO: 11 and the VH CDR3 sequence set forth in SEQ ID NO:12 and the second antigen-binding region comprises the VH CDR1 sequence set forth in SEQ ID NO:34, the VH CDR2 sequence set forth in SEQ ID NO:35 and the VH CDR3 sequence set forth in SEQ ID NO:36, or
(iii) the first antigen-binding region comprises the VH CDR1 sequence set forth in SEQ ID NO:18, the VH CDR2 sequence set forth in SEQ ID NO:19 and the VH CDR3 sequence set forth in SEQ ID NO:20 and the second antigen-binding region comprises the VH CDR1 sequence set forth in SEQ ID NO:34, the VH CDR2 sequence set forth in SEQ ID NO:35 and the VH CDR3 sequence set forth in SEQ ID NO:36, or
(iv) the first antigen-binding region comprises the VH CDR1 sequence set forth in SEQ ID NO:26, the VH CDR2 sequence set forth in SEQ ID NO:27 and the VH CDR3 sequence set forth in SEQ ID NO:28 and the second antigen-binding region comprises the VH CDR1 sequence set forth in SEQ ID NO:34, the VH CDR2 sequence set forth in SEQ ID NO:35 and the VH CDR3 sequence set forth in SEQ ID NO:36.

In one embodiment of the multispecific antibody of the invention,
(i) the first antigen-binding region comprises the VH CDR1 sequence set forth in SEQ ID NO:2, the VH CDR2 sequence set forth in SEQ ID NO:3 and the VH CDR3 sequence set forth in SEQ ID NO:4 and the second antigen-binding region comprises the VH CDR1 sequence set forth in SEQ ID NO:34, the VH CDR2 sequence set forth in SEQ ID NO:35 and the VH CDR3 sequence set forth in SEQ ID NO:36 wherein X₁ is S, X₂ is F and X₃ is A, or
(ii) the first antigen-binding region comprises the VH CDR1 sequence set forth in SEQ ID NO:10, the VH CDR2 sequence set forth in SEQ ID NO:11 and the VH CDR3 sequence set forth in SEQ ID NO: 12 and the second antigen-binding region comprises the VH CDR1 sequence set forth in SEQ ID NO:34, the VH CDR2 sequence set forth in SEQ ID NO:35 and the VH CDR3 sequence set forth in SEQ ID NO:36 wherein X₁ is S, X₂ is F and X₃ is A, or
(iii) the first antigen-binding region comprises the VH CDR1 sequence set forth in SEQ ID NO:18, the VH CDR2 sequence set forth in SEQ ID NO:19 and the VH CDR3 sequence set forth in SEQ ID NO:20 and the second antigen-binding region comprises the VH CDR1 sequence set forth in SEQ ID NO:34, the VH CDR2 sequence set forth in SEQ ID NO:35 and the VH CDR3 sequence set forth in SEQ ID NO:36 wherein X₁ is S, X₂ is F and X₃ is A, or
(iv) the first antigen-binding region comprises the VH CDR1 sequence set forth in SEQ ID NO:26, the VH CDR2 sequence set forth in SEQ ID NO:27 and the VH CDR3 sequence set forth in SEQ ID NO:28 and the second antigen-binding region comprises the VH CDR1 sequence set forth in SEQ ID NO:34, the VH CDR2 sequence set forth in SEQ ID NO:35 and the VH CDR3 sequence set forth in SEQ ID NO:36 wherein X₁ is S, X₂ is F and X₃ is A.

In one embodiment of the multispecific antibody of the invention,
(i) the first antigen-binding region comprises the VH CDR1 sequence set forth in SEQ ID NO:2, the VH CDR2 sequence set forth in SEQ ID NO:3 and the VH CDR3 sequence set forth in SEQ ID NO:4 and the second antigen-binding region comprises the VH CDR1 sequence set forth in SEQ ID NO:39, the VH CDR2 sequence set forth in SEQ ID NO:40 and the VH CDR3 sequence set forth in SEQ ID NO:41, or
(ii) the first antigen-binding region comprises the VH CDR1 sequence set forth in SEQ ID NO:10, the VH CDR2 sequence set forth in SEQ ID NO: 11 and the VH CDR3 sequence set forth in SEQ ID NO: 12 and the second antigen-binding region comprises the VH CDR1 sequence set forth in SEQ ID NO:39, the VH CDR2 sequence set forth in SEQ ID NO:40 and the VH CDR3 sequence set forth in SEQ ID NO:41, or
(iii) the first antigen-binding region comprises the VH CDR1 sequence set forth in SEQ ID NO:18, the VH CDR2 sequence set forth in SEQ ID NO:19 and the VH CDR3 sequence set forth in SEQ ID NO:20 and the second antigen-binding region comprises the VH CDR1 sequence set forth in SEQ ID NO:39, the VH CDR2 sequence set forth in SEQ ID NO:40 and the VH CDR3 sequence set forth in SEQ ID NO:41, or
(iv) the first antigen-binding region comprises the VH CDR1 sequence set forth in SEQ ID NO:26, the VH CDR2 sequence set forth in SEQ ID NO:27 and the VH CDR3 sequence set forth in SEQ ID NO:28 and the second antigen-binding region comprises the VH CDR1 sequence set forth in SEQ ID NO:39, the VH CDR2 sequence set forth in SEQ ID NO:40 and the VH CDR3 sequence set forth in SEQ ID NO:41.

In further preferred embodiments of the multispecific antibody of the invention,
(i) the first antigen-binding region comprises or consists of the sequence set forth in SEQ ID NO: 1 and the second antigen-binding region comprises or consists of the sequence set forth in SEQ ID NO:33, or
(ii) the first antigen-binding region comprises or consists of the sequence set forth in SEQ ID NO:9 and the second antigen-binding region comprises or consists of the sequence set forth in SEQ ID NO:33, or
(iii) the first antigen-binding region comprises or consists of the sequence set forth in SEQ ID NO:17 and the second antigen-binding region comprises or consists of the sequence set forth in SEQ ID NO:33, or
(iv) the first antigen-binding region comprises or consists of the sequence set forth in SEQ ID NO:25 and the second antigen-binding region comprises or consists of the sequence set forth in SEQ ID NO:33, or
(v) the first antigen-binding region comprises or consists of the sequence set forth in SEQ ID NO: 1 and the second antigen-binding region comprises or consists of the sequence set forth in SEQ ID NO:37, or
(vi) the first antigen-binding region comprises or consists of the sequence set forth in SEQ ID NO:9 and the second antigen-binding region comprises or consists of the sequence set forth in SEQ ID NO:37, or
(vii) the first antigen-binding region comprises or consists of the sequence set forth in SEQ ID NO:17 and the second antigen-binding region comprises or consists of the sequence set forth in SEQ ID NO:37, or
(viii) the first antigen-binding region comprises or consists of the sequence set forth in SEQ ID NO:25 and the second antigen-binding region comprises or consists of the sequence set forth in SEQ ID NO:37, or
(ix) the first antigen-binding region comprises or consists of the sequence set forth in SEQ ID NO: 1 and the second antigen-binding region comprises or consists of the sequence set forth in SEQ ID NO:38, or
(x) the first antigen-binding region comprises or consists of the sequence set forth in SEQ ID NO:9 and the second antigen-binding region comprises or consists of the sequence set forth in SEQ ID NO:38, or
(xi) the first antigen-binding region comprises or consists of the sequence set forth in SEQ ID NO:17 and the second antigen-binding region comprises or consists of the sequence set forth in SEQ ID NO:38, or
(xii) the first antigen-binding region comprises or consists of the sequence set forth in SEQ ID NO:25 and the second antigen-binding region comprises or consists of the sequence set forth in SEQ ID NO:38.

The first and second antigen-binding regions in the multispecific antibody may be arranged in various ways. In one embodiment, antigen-binding regions are connected to each other via a linker, such as a covalent linker. In one embodiment, the first antigen-binding region and the second antigen-binding region are covalently linked to each other via a peptide linker, e.g. a linker having a length of from 1 to 20 amino acids, e.g. from 1 to 10 amino acids, such as 2, 3, 4, 5, 6, 7, 8, 9 or 10 amino acids. In one embodiment, the peptide linker comprises or consists of a sequence of 4 glycines following by a serine.

In some embodiments, the first antigen-binding region capable of binding human Nectin-4 is located N-terminally of the second antigen-binding region capable of binding the human Vγ9Vδ2 T cell receptor. In another embodiment, the first antigen-binding region capable of binding human Nectin-4 is located C-terminally of the second antigen-binding region capable of binding the human Vγ9Vδ2 T cell receptor.

Multispecific antibodies of the invention, such as bispecific antibodies, may contain further molecules, domains or polypeptide sequences beyond the first and second antigen-binding regions. In one embodiment, the multispecific antibody further comprises a half-life extension domain, i.e. a domain which prolongs the half-life of the molecules in the circulation of a human patient. In one embodiment, the multispecific antibody has a terminal half-life that is longer than about 168 hours when administered to a human subject. Most preferably the terminal half-life is 336 hours or longer. The "terminal half-life" of an antibody, when used herein refers to the time taken for the serum concentration of the polypeptide to be reduced by 50%, in vivo in the final phase of elimination.

In one embodiment, the multispecific antibody comprises an Fc region, preferably a human Fc region. In one embodiment, the multispecific antibody is in a VHH-Fc format, i.e. the antibody comprises two or more single-domain antigen-binding regions that are linked to each other via a human Fc region dimer, wherein each single-domain antigen-binding region is fused to an Fc region polypeptide (without CH1 or light chain sequences) and the two fusion polypeptides form a dimeric bispecific antibody via disulfide bridges in the hinge region.

Various methods for making multispecific, such as bispecific, antibodies have been described in the art, e.g. reviewed by Brinkmann and Kontermann (2017) MAbs 9:182 and Labrijn et al (2019) Nature Reviews Drug Discovery 18: 585. In one embodiment, the Fc region is a heterodimer comprising two Fc polypeptides, wherein the first antigen-binding region is fused to the first Fc polypeptide and the second antigen-binding region is fused to the second Fc polypeptide and wherein the first and second Fc polypeptides comprise asymmetric amino acid mutations that favor the formation of heterodimers over the formation of homodimers (see e.g. Ridgway et al. (1996) 'Knobs-into-holes' engineering of antibody CH3 domains for heavy chain heterodimerization. Protein Eng 9:617). In a further embodiment hereof, the CH3 regions of the Fc polypeptides comprise said asymmetric amino acid mutations, preferably the first Fc polypeptide comprises a T366W substitution and the second Fc polypeptide comprises T366S, L368A and Y407V substitutions, or vice versa, wherein the amino acid positions correspond to human IgG1 according to the EU numbering system.

In one embodiment, the cysteine residues at position 220 in the first and second Fc polypeptides have been deleted or substituted, wherein the amino acid position corresponds to human IgG1 according to the EU numbering system. In a further embodiment, the region comprises the sequence set forth in SEQ ID NO:46.

In one embodiment, the first and/or second Fc polypeptides contain mutations that render the Fc region inert, i.e. unable to mediate effector functions. In one embodiment, the first and second Fc polypeptides comprise a mutation at position 234 and/or 235, preferably the first and second Fc polypeptide comprise an L234F and an L235E substitution, wherein the amino acid positions correspond to human IgG1 according to the EU numbering system.

In one embodiment, the first Fc polypeptide comprises the sequence set forth in SEQ ID NO:42 and the second Fc polypeptide comprises the sequence set forth in SEQ ID NO:43, or vice versa.

In a further preferred embodiment, the multispecific antibody of the invention consists of:
(i) a first polypeptide chain consisting of: a first antigen-binding region consisting of the sequence set forth in SEQ ID NO:1, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:33, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO:1, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:33, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO:1, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:37, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO:1, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:37, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO:1, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:38, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO:1, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:38, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO:9, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:33, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO:9, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:33, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO:9, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:37, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO:9, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:37, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO:9, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:38, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO:9, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:38, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO: 17, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:33, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO: 17, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:33, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO: 17, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:37, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO: 17, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:37, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO: 17, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:38, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO:17, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:38, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO:25, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:33, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO:25, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:33, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO:25, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:37, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO:25, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:37, the sequence set forth in SEQ ID NO:46, and the sequence set forth in SEQ ID NO:42.

In a further preferred embodiment, the multispecific antibody of the invention consists of:
(i) a first polypeptide chain consisting of: a first antigen-binding region consisting of the sequence set forth in SEQ ID NO:25, the sequence set forth in SEQ ID NO:46, and the sequence set forth in SEQ ID NO:42, and
(ii) a second polypeptide chain consisting of: a second antigen-binding region consisting of the sequence set forth in SEQ ID NO:38, the sequence set forth in SEQ ID NO:46, and the sequence set forth in SEQ ID NO:43.

In a further preferred embodiment, the multispecific antibody of the invention consists of:
(i) a first polypeptide chain consisting of: a first antigen-binding region consisting of the sequence set forth in SEQ ID NO:25, the sequence set forth in SEQ ID NO:46, and the sequence set forth in SEQ ID NO:43, and
(ii) a second polypeptide chain consisting of: a second antigen-binding region consisting of the sequence set forth in SEQ ID NO:38, the sequence set forth in SEQ ID NO:46, and the sequence set forth in SEQ ID NO:42.

In a further preferred embodiment, the multispecific antibody of the invention comprises or consists of the polypeptide set forth in SEQ ID NO:48 and the polypeptide set forth in SEQ ID NO:52.

In a further preferred embodiment, the multispecific antibody of the invention comprises or consists of the polypeptide set forth in SEQ ID NO:49 and the polypeptide set forth in SEQ ID NO:52.

In a further preferred embodiment, the multispecific antibody of the invention comprises or consists of the polypeptide set forth in SEQ ID NO:50 and the polypeptide set forth in SEQ ID NO:52.

In a further preferred embodiment, the multispecific antibody of the invention comprises or consists of the polypeptide set forth in SEQ ID NO:51 and the polypeptide set forth in SEQ ID NO:52.

In one embodiment, the multispecific antibody is capable of binding to recombinant Nectin-4 protein with a K_{D} of less than 10 nM, for example less than 5 nM, less than 2 nM, less than 1 nM or less than 0.5 nM, as determined by Bio-Layer Interferometry according to the methods described in Example 5 herein.

In one embodiment, the multispecific antibody is capable of binding to recombinant Nectin-4 protein with a K_{D} of between 10 nM and 1 nM, such as between 5 nM and 1 nM. as determined by Bio-Layer Interferometry according to the methods described in Example 5 herein.

In one embodiment, the multispecific antibody is capable of binding to BeWo cells with an EC50 of less than 0.5 nM, when tested as described in Example 3 herein.

In one embodiment, the multispecific antibody is capable of binding to A-431 cells with an EC50 of less than 0.5 nM, when tested as described in Example 3 herein.

In one embodiment, the multispecific antibody is capable of binding to HT-29 cells with an EC50 of less than 2 nM, when tested as described in Example 3 herein.

In one embodiment, the multispecific antibody is capable of binding to Vγ9Vδ2 T cells with an EC50 of less than 5 nM, such as less than 2 nM, when tested as described in Example 9 herein.

In one embodiment, the multispecific antibody is capable of binding cynomolgus Nectin-4 and/or murine Nectin-4.

In one embodiment, the multispecific antibody is capable of mediating killing of Nectin-4-expressing tumor cells, such as BeWo, A-431 or HT-29 cells, by Vγ9Vδ2 T cells.

Preferably, the antibody is capable of inducing killing of A-431 cells through activation of Vγ9Vδ2 T cells with an EC50 value of 100 pM or less, such as 20 pM or less, e.g. 10 pM or less, or even 7 pM or less, or 5 pM or less when tested as described in Example 10 herein.

In a further embodiment, the multispecific antibody is not capable of mediating killing of Nectin-4-negative cells, such as Nectin-4 negative human cells.

In a further embodiment, the multispecific antibody:
- is capable of binding to recombinant human Nectin-4 with a K_{D} of less than 10 nM, for example less than 5 nM, less than 2 nM, less than 1 nM or less than 0.5 nM, as determined by Bio-Layer Interferometry according to the methods described in Example 5 herein, and
- is capable of inducing killing of A-431 cells through activation of Vγ9Vδ2 T cells with an EC50 value of 10 pM or less, or even 7 pM or less, or 5 pM or less when tested as described in Example 10 herein.

In a further embodiment, the multispecific antibody:
- is capable of binding to recombinant Nectin-4 protein with a K_{D} of between 10 nM and 1 nM, such as between 5 nM and 1 nM. as determined by Bio-Layer Interferometry according to the methods described in Example 5 herein, and
- is capable of inducing killing of A-431 cells through activation of Vγ9Vδ2 T cells with an EC50 value of 10 pM or less, or even 7 pM or less, or 5 pM or less when tested as described in Example 10 herein.

In a further embodiment, the multispecific antibody has all of the following properties:
- is capable of mediating killing BeWo, A-431 and HT-29 cells, by Vγ9Vδ2 T cells,
- is capable of inducing killing of A-431 cells through activation of Vγ9Vδ2 T cells with an EC50 value of 100 pM or less, such as 20 pM or less, e.g. 10 pM or less, or even 7 pM or less, or 5 pM or less when tested as described in Example 10 herein.
- is capable of binding human Nectin-4, cynomolgous Nectin4 and murine Nectin-4, and
- is capable of binding to recombinant human Nectin-4 with a K_{D} of less than 10 nM, for example less than 5 nM, less than 2 nM, less than 1 nM or less than 0.5 nM, as determined by Bio-Layer Interferometry according to the methods described in Example 5 herein.

In a further embodiment, the multispecific antibody has all of the following properties:
- is capable of mediating killing BeWo, A-431 and HT-29 cells, by Vγ9Vδ2 T cells,
- is capable of inducing killing of A-431 cells through activation of Vγ9Vδ2 T cells with an EC50 value of 100 pM or less, such as 20 pM or less, e.g. 10 pM or less, or even 7 pM or less, or 5 pM or less when tested as described in Example 10 herein,
- is capable of binding human Nectin-4, cynomolgous Nectin4 and murine Nectin-4, and
- is capable of binding to recombinant Nectin-4 protein with a K_{D} of between 10 nM and 1 nM, such as between 5 nM and 1 nM. as determined by Bio-Layer Interferometry according to the methods described in Example 5 herein.

In a further main aspect, the invention relates to an antibody comprising a first antigen-binding region capable of binding human Nectin-4, wherein the antibody is capable of competing for binding to human Nectin-4 with an antibody having the sequence set forth in SEQ ID NO: 1.

The antibody may or may not further comprise additional antigen-binding regions. Thus, the antibody may be monospecific or multispecific. Furthermore, the antibody may be monovalent or multivalent. Additional antigen-binding regions, if present, may bind to other targets than Nectin-4. In one embodiment, said first antigen-binding region is a single-domain antibody.

In a further aspect, the invention provides an antibody comprising a first antigen-binding region capable of binding human Nectin-4, wherein the antibody is capable of competing for binding to human Nectin-4 with an antibody having the sequence set forth in SEQ ID NO:9.

In a further aspect, the invention provides an antibody comprising a first antigen-binding region capable of binding human Nectin-4, wherein the antibody is capable of competing for binding to human Nectin-4 with an antibody having the sequence set forth in SEQ ID NO:17.

In a further aspect, the invention provides an antibody comprising a first antigen-binding region capable of binding human Nectin-4, wherein the antibody is capable of competing for binding to human Nectin-4 with an antibody having the sequence set forth in SEQ ID NO:25.

In one embodiment, the first antigen-binding region is a single-domain antibody comprising:
(i) the CDR1 sequence set forth in SEQ ID NO:2, the CDR2 sequence set forth in SEQ ID NO:3 and the CDR3 sequence set forth in SEQ ID NO:4, or a CDR1 sequence which differs from the CDR1 sequence set forth in SEQ ID NO:2 in one or two positions, a CDR2 sequence which differs from the CDR2 sequence set forth in SEQ ID NO:3 in one, two, three, four or five positions and a CDR3 sequence which differs from the CDR3 sequence set forth in SEQ ID NO:4 in one or two positions,
(ii) the CDR1 sequence set forth in SEQ ID NO:10, the CDR2 sequence set forth in SEQ ID NO:11 and the CDR3 sequence set forth in SEQ ID NO:12, or a CDR1 sequence which differs from the CDR1 sequence set forth in SEQ ID NO:10 in one or two positions, a CDR2 sequence which differs from the CDR2 sequence set forth in SEQ ID NO:11 in one, two, three, four or five positions and a CDR3 sequence which differs from the CDR3 sequence set forth in SEQ ID NO:12 in one or two positions,
(iii) the CDR1 sequence set forth in SEQ ID NO:18, the CDR2 sequence set forth in SEQ ID NO:19 and the CDR3 sequence set forth in SEQ ID NO:20, or a CDR1 sequence which differs from the CDR1 sequence set forth in SEQ ID NO:18 in one or two positions, a CDR2 sequence which differs from the CDR2 sequence set forth in SEQ ID NO:19 in one, two, three, four or five positions and a CDR3 sequence which differs from the CDR3 sequence set forth in SEQ ID NO:20 in one or two positions, or
(iv) the CDR1 sequence set forth in SEQ ID NO:26, the CDR2 sequence set forth in SEQ ID NO:27 and the CDR3 sequence set forth in SEQ ID NO:28, or a CDR1 sequence which differs from the CDR1 sequence set forth in SEQ ID NO:26 in one or two positions, a CDR2 sequence which differs from the CDR2 sequence set forth in SEQ ID NO:27 in one, two, three, four or five positions and a CDR3 sequence which differs from the CDR3 sequence set forth in SEQ ID NO:28 in one or two positions, or
(v) the CDR1 sequence set forth in SEQ ID NO:66, the CDR2 sequence set forth in SEQ ID NO:67 and the CDR3 sequence set forth in SEQ ID NO:68, or a CDR1 sequence which differs from the CDR1 sequence set forth in SEQ ID NO:66 in one or two positions, a CDR2 sequence which differs from the CDR2 sequence set forth in SEQ ID NO:67 in one, two, three, four or five positions and a CDR3 sequence which differs from the CDR3 sequence set forth in SEQ ID NO:68 in one or two positions, or
(vi) the CDR1 sequence set forth in SEQ ID NO:70, the CDR2 sequence set forth in SEQ ID NO:71 and the CDR3 sequence set forth in SEQ ID NO:72, or a CDR1 sequence which differs from the CDR1 sequence set forth in SEQ ID NO:70 in one or two positions, a CDR2 sequence which differs from the CDR2 sequence set forth in SEQ ID NO:71 in one, two, three, four or five positions and a CDR3 sequence which differs from the CDR3 sequence set forth in SEQ ID NO:72 in one or two positions, or
(vii) the CDR1 sequence set forth in SEQ ID NO:74, the CDR2 sequence set forth in SEQ ID NO:75 and the CDR3 sequence set forth in SEQ ID NO:76, or a CDR1 sequence which differs from the CDR1 sequence set forth in SEQ ID NO:74 in one or two positions, a CDR2 sequence which differs from the CDR2 sequence set forth in SEQ ID NO:75 in one, two, three, four or five positions and a CDR3 sequence which differs from the CDR3 sequence set forth in SEQ ID NO:76 in one or two positions.

In one embodiment, the first antigen-binding region comprises or consists of: the sequence set forth in SEQ ID NO:1, or a sequence having at least 90%, such as least 92%, e.g. at least 94%, such as at least 96%, e.g. at least 98% sequence identity to the sequence set forth in SEQ ID NO:1.

In one embodiment, the first antigen-binding region comprises or consists of the sequence set forth in SEQ ID NO:1.

In one embodiment of the multispecific antibody of the invention, the first antigen-binding region comprises the CDR1 sequence set forth in SEQ ID NO:2, the CDR2 sequence set forth in SEQ ID NO:3 and the CDR3 sequence set forth in SEQ ID NO:4 and comprises or consists of a sequence having at least 90%, such as least 92%, e.g. at least 94%, such as at least 96%, e.g. at least 98% sequence identity to the sequence ser forth in SEQ ID NO:1.

In one embodiment, the first antigen-binding region comprises or consists of: the sequence set forth in SEQ ID NO:9, or a sequence having at least 90%, such as least 92%, e.g. at least 94%, such as at least 96%, e.g. at least 98% sequence identity to the sequence set forth in SEQ ID NO:9.

In one embodiment, the first antigen-binding region comprises or consists of the sequence set forth in SEQ ID NO:9.

In one embodiment of the multispecific antibody of the invention, the first antigen-binding region comprises the CDR1 sequence set forth in SEQ ID NO: 10, the CDR2 sequence set forth in SEQ ID NO: 11 and the CDR3 sequence set forth in SEQ ID NO:12 and comprises or consists of a sequence having at least 90%, such as least 92%, e.g. at least 94%, such as at least 96%, e.g. at least 98% sequence identity to the sequence ser forth in SEQ ID NO:9.

In one embodiment, the first antigen-binding region comprises or consists of: the sequence set forth in SEQ ID NO:17, or a sequence having at least 90%, such as least 92%, e.g. at least 94%, such as at least 96%, e.g. at least 98% sequence identity to the sequence set forth in SEQ ID NO:17.

In one embodiment, the first antigen-binding region comprises or consists of the sequence set forth in SEQ ID NO:17.

In one embodiment of the multispecific antibody of the invention, the first antigen-binding region comprises the CDR1 sequence set forth in SEQ ID NO:18, the CDR2 sequence set forth in SEQ ID NO:19 and the CDR3 sequence set forth in SEQ ID NO:20 and comprises or consists of a sequence having at least 90%, such as least 92%, e.g. at least 94%, such as at least 96%, e.g. at least 98% sequence identity to the sequence ser forth in SEQ ID NO:17.

In one embodiment, the first antigen-binding region comprises or consists of: the sequence set forth in SEQ ID NO:25, or a sequence having at least 90%, such as least 92%, e.g. at least 94%, such as at least 96%, e.g. at least 98% sequence identity to the sequence set forth in SEQ ID NO:25.

In one embodiment, the first antigen-binding region comprises or consists of the sequence set forth in SEQ ID NO:25.

In one embodiment of the multispecific antibody of the invention, the first antigen-binding region comprises the CDR1 sequence set forth in SEQ ID NO:26, the CDR2 sequence set forth in SEQ ID NO:27 and the CDR3 sequence set forth in SEQ ID NO:28 and comprises or consists of a sequence having at least 90%, such as least 92%, e.g. at least 94%, such as at least 96%, e.g. at least 98% sequence identity to the sequence ser forth in SEQ ID NO:25.

In one embodiment, the first antigen-binding region comprises or consists of: the sequence set forth in SEQ ID NO:65, or a sequence having at least 90%, such as least 92%, e.g. at least 94%, such as at least 96%, e.g. at least 98% sequence identity to the sequence set forth in SEQ ID NO:65.

In one embodiment, the first antigen-binding region comprises or consists of: the sequence set forth in SEQ ID NO:69, or a sequence having at least 90%, such as least 92%, e.g. at least 94%, such as at least 96%, e.g. at least 98% sequence identity to the sequence set forth in SEQ ID NO:69.

In one embodiment, the first antigen-binding region comprises or consists of: the sequence set forth in SEQ ID NO:73, or a sequence having at least 90%, such as least 92%, e.g. at least 94%, such as at least 96%, e.g. at least 98% sequence identity to the sequence set forth in SEQ ID NO:73.

In a further main aspect, the invention relates to a pharmaceutical composition comprising an antibody, such as a multispecific antibody, according to the invention as described herein and a pharmaceutically-acceptable excipient.

Antibodies may be formulated with pharmaceutically-acceptable excipients in accordance with conventional techniques such as those disclosed in (Rowe et al., Handbook of Pharmaceutical Excipients, 2012 June, ISBN 9780857110275). The pharmaceutically-acceptable excipient as well as any other carriers, diluents or adjuvants should be suitable for the antibodies and the chosen mode of administration. Suitability for excipients and other components of pharmaceutical compositions is determined based on the lack of significant negative impact on the desired biological properties of the chosen antibody or pharmaceutical composition of the present invention (e.g., less than a substantial impact (10% or less relative inhibition, 5% or less relative inhibition, etc.) upon antigen binding).

A pharmaceutical composition may include diluents, fillers, salts, buffers, detergents (e.g., a nonionic detergent, such as Tween-20 or Tween-80), stabilizers (e.g., sugars or protein-free amino acids), preservatives, tissue fixatives, solubilizers, and/or other materials suitable for inclusion in a pharmaceutical composition. Further pharmaceutically-acceptable excipients include any and all suitable solvents, dispersion media, coatings, antibacterial and antifungal agents, isotonicity agents, antioxidants and absorption-delaying agents and the like that are physiologically compatible with an antibody of the present invention.

In a further main aspect, the invention relates to an antibody, such as a multispecific antibody, according to the invention as described herein for use as a medicament.

A multispecific antibody according to the invention enables killing of tumor cells, in particular Nectin-4-positive tumor cells, by Vγ9Vδ2 T cells.

Accordingly, in a further main aspect, the invention relates to an antibody, such as a multispecific antibody, according to the invention as described herein for use in the treatment of cancer. In a further main aspect, the invention relates to an antibody, such as a multispecific antibody according to the invention as described herein for use in the treatment of cancer, such as bladder cancer (such as bladder urothelial cancer), breast cancer (such as triple negative breast cancer), renal cancer, prostate cancer, ovarian cancer, esophageal cancer, head and neck cancer, lung cancer, pancreatic cancer, gastric cancer, thyroid cancer, colorectal cancer, cholangiocarcinoma or uterine corpus endometrial carcinoma.

Similarly, the invention relates to a method of treating a disease comprising administration of a multispecific antibody according to the invention as described herein to a human subject in need thereof. In one embodiment, the disease is cancer, such as bladder cancer (such as bladder urothelial cancer), breast cancer (such as triple negative breast cancer), renal cancer, prostate cancer, ovarian cancer, esophageal cancer, head and neck cancer, lung cancer, pancreatic cancer, gastric cancer, thyroid cancer, colorectal cancer, cholangiocarcinoma or uterine corpus endometrial carcinoma.

In some embodiments, the antibody is administered as monotherapy. However, antibodies of the present invention may also be administered in combination therapy, i.e., combined with other therapeutic agents relevant for the disease or condition to be treated.

"Treatment" or "treating" refers to the administration of an effective amount of an antibody according to the present invention with the purpose of easing, ameliorating, arresting, eradicating (curing) or preventing symptoms or disease states. An "effective amount" refers to an amount effective, at dosages and for periods of time necessary, to achieve a desired therapeutic result. An effective amount of a polypeptide, such as an antibody, may vary according to factors such as the disease stage, age, sex, and weight of the individual, and the ability of the antibody to elicit a desired response in the individual. An effective amount is also one in which any toxic or detrimental effects of the antibody are outweighed by the therapeutically beneficial effects. Administration may be carried out by any suitable route, but will typically be parenteral.

Multispecific antibodies of the invention are typically produced recombinantly, i.e. by expression of nucleic acid constructs encoding the antibodies in suitable host cells, followed by purification of the produced recombinant antibody from the cell culture. Nucleic acid constructs can be produced by standard molecular biological techniques well-known in the art. The constructs are typically introduced into the host cell using an expression vector. Suitable nucleic acid constructs and expression vectors are known in the art. Host cells suitable for the recombinant expression of antibodies are well-known in the art, and include CHO (Chinese Hamster Ovary), HEK-293, Expi293F, PER-C6, NS/0 and Sp2/0 cells.

Accordingly, in a further aspect, the invention relates to a nucleic acid construct encoding an antibody of the invention, such as multispecific antibody according to the invention. In one embodiment, the construct is a DNA construct. In another embodiment, the construct is an RNA construct. The nucleic acid construct typically further comprises a promoter.

In a further aspect, the invention relates to an expression vector comprising a nucleic acid construct encoding a multispecific antibody according to the invention. The expression vector typically further comprises a promoter.

In a further aspect, the invention relates to a host cell, such as a non-human host cell, for example a CHO cell, comprising one or more nucleic acid constructs encoding a multispecific antibody according to the invention or an expression vector comprising a nucleic acid construct encoding a multispecific antibody according to the invention.

**Table 1: Sequence listing. CDR sequences were determined according to Kabat et al., supra.**

| SEQ ID. | code | Description | Sequence |
|---|---|---|---|
| 1 | LV1184 | VHH | EVQLVESGGGLVQAGGSLRLSCAASGSISSINLM GWFRQAPAKQRELVTSISPGGSVRYADSVKGRF TISRDVAKNTVDLQMNSLKPEDTAVYYCAAESER TYYFDSWGQGTQVTVSS |
| 2 | LV1184 | CDR1 | INLMG |
| 3 | LV1184 | CDR2 | SISPGGSVRYADSVKG |
| 4 | LV1184 | CDR3 | ESERTYYFDS |
| 5 | LV1184 | FR1 | EVQLVESGGGLVQAGGSLRLSCAASGSISS |
| 6 | LV1184 | FR2 | WFRQAPAKQRELVT |
| 7 | LV1184 | FR3 | RFTISRDVAKNTVDLQMNSLKPEDTAVYYCAA |
| 8 | LV1184 | FR4 | WGQGTQVTVSS |
| 9 | LV1181 | VHH | EVQLVESGGGLVQAGGSLRLSCAASGSISSINL MGWHRQAPGKQRELVATITRGGSTNYADSVKG RFTISRDNGKNTVYLQMNSLKPEDTAAYYCVDVE PSGMGWRDYWGQGTQVTVSS |
| 10 | LV1181 | CDR1 | INLMG |
| 11 | LV1181 | CDR2 | TITRGGSTNYADSVKG |
| 12 | LV1181 | CDR3 | VEPSGMGWRDY |
| 13 | LV1181 | FR1 | EVQLVESGGGLVQAGGSLRLSCAASGSISS |
| 14 | LV1181 | FR2 | WHRQAPGKQRELVA |
| 15 | LV1181 | FR3 | RFTISRDNGKNTVYLQMNSLKPEDTAAYYCVD |
| 16 | LV1181 | FR4 | WGQGTQVTVSS |
| 17 | LV1185 | VHH | EVQLVESGGGLVQAGGSLRLSCAASRSTFSLNIM GWYRQAPGKQREYVATITTGGSTNYADSVRGRF TISRDNAEDTVYLQMNSLKPEDTAVYYCTAELVR RGPTTYWGRGTQVTVSS |
| 18 | LV1185 | CDR1 | LNIMG |
| 19 | LV1185 | CDR2 | TITTGGSTNYADSVRG |
| 20 | LV1185 | CDR3 | ELVRRGPTTY |
| 21 | LV1185 | FR1 | EVQLVESGGGLVQAGGSLRLSCAASRSTFS |
| 22 | LV1185 | FR2 | WYRQAPGKQREYVA |
| 23 | LV1185 | FR3 | RFTISRDNAEDTVYLQMNSLKPEDTAVYYCTA |
| 24 | LV1185 | FR4 | WGRGTQVTVSS |
| 25 | LV1186 | VHH | EVQLVESGGGLVQAGGSLRLSCAASRSTFSLNIM GWYRQAPGKQREYVATITSGGSTNYADSVRGRF TISRDNAENTVYLQMDSLKPEDTAVYYCTAELVR RGPTTYWGRGTQVTVSS |
| 26 | LV1186 | CDR1 | LNIMG |
| 27 | LV1186 | CDR2 | TITSGGSTNYADSVRG |
| 28 | LV1186 | CDR3 | ELVRRGPTTY |
| 29 | LV1186 | FR1 | EVQLVESGGGLVQAGGSLRLSCAASRSTFS |
| 30 | LV1186 | FR2 | WYRQAPGKQREYVA |
| 31 | LV1186 | FR3 | RFTISRDNAENTVYLQMDSLKPEDTAVYYCTA |
| 32 | LV1186 | FR4 | WGRGTQVTVSS |
| 33 | 5C8var | VHH | EVQLLESGGGSVQPGGSLRLSCAASGRPFSNYA MX1WFRQAPGKEREFVSAISWSGGSTSYADSVK GRFTISRDNSKNTLYLQMNSLRAEDTAVYYCAA QFSGADX2GFGX3LGIRGYEYDYWGQGTQVTVS |
| | | | S, wherein X₁ is S or G, wherein X₂ can be any amino acid and wherein X₃ is not R |
| 34 | 5C8var | CDR1 | NYAMX₁, wherein X₁ is S or G |
| 35 | 5C8var | CDR2 | AISWSGGSTSYADSVKG |
| 36 | 5C8var | CDR3 | QFSGADX₂GFGX₃LGIRGYEDY, wherein X₂ can be any amino acid and wherein X₃ is not R |
| 37 | 5C8 variant | VHH | EVQLLESGGGSVQPGGSLRLSCAASGRPFSNYA MSWFRQAPGKEREFVSAISWSGGSTSYADSVK GRFTISRDNSKNTLYLQMNSLRAEDTAVYYCAA QFSGADFGFGALGIRGYEYDYWGQGTQVTVSS |
| 38 | 6H4 | VHH | EVQLVESGGGLVQAGGSLRLSCAASGRPFSNYG MGWFRQAPGKKREFVAGISWSGGSTDYADSVK GRFTISRDNAKNTVYLQMNSLKPEDTAVYYCAAV FSGAETAYYPSDDYDYWGQGTQVTVSS |
| 39 | 6H4 | CDR1 | NYGMG |
| 40 | 6H4 | CDR2 | GISWSGGSTDYADSVKG |
| 41 | 6H4 | CDR3 | VFSGAETAYYPSDDYDY |
| 42 | KiH (hole) LFLE | Fc | APEFEGGPSVFLFPPKPKDTLMISRTPEVTCVVVD VSHEDPEVKFNWYVDGVEVHNAKTKPREEQYNS TYRVVSVLTVLHQDWLNGKEYKCKVSNKALPAPI EKTISKAKGQPREPQVYTLPPSRDELTKNQVSLS CAVKGFYPSDIAVEWESNGQPENNYKTTPPVLDS DGSFFLVSKLTVDKSRWQQGNVFSCSVMHEALH NHYTQKSLSLSPGX4 |
| | | | wherein X₄ is a K that may or may not be present |
| 43 | KiH (knob) LFLE | Fc | APEFEGGPSVFLFPPKPKDTLMISRTPEVTCVVVD VSHEDPEVKFNWYVDGVEVHNAKTKPREEQYNS TYRVVSVLTVLHQDWLNGKEYKCKVSNKALPAPI EKTISKAKGQPREPQVYTLPPSRDELTKNQVSLW CLVKGFYPSDIAVEWESNGQPENNYKTTPPVLDS DGSFFLYSKLTVDKSRWQQGNVFSCSVMHEALH NHYTQKSLSLSPGX4 |
| | | | wherein X₄ is a K that may or may not be present |
| 44 | Human Nectin-4 | | MPLSLGAEMW GPEAWLLLLL LLASFTGRCP AGELETSDVV TVVLGQDAKL PCFYRGDSGE QVGQVAWARV DAGEGAQELA LLHSKYGLHV SPAYEGRVEQ PPPPRNPLDG SVLLRNAVQA DEGEYECRVS TFPAGSFQAR LRLRVLVPPL PSLNPGPALE EGQGLTLAAS CTAEGSPAPS VTWDTEVKGT TSSRSFKHSR SAAVTSEFHL VPSRSMNGQP LTCVVSHPGL LQDQRITHIL HVSFLAEASV RGLEDQNLWH IGREGAMLKC LSEGQPPPSY NWTRLDGPLP SGVRVDGDTL GFPPLTTEHS GIYVCHVSNE FSSRDSQVTV DVLDPQEDSG KQVDLVSASV VVVGVIAALL FCLLVVVVVL MSRYHRRKAQ QMTQKYEEEL TLTRENSIRR LHSHHTDPRS QPEESVGLRA EGHPDSLKDN SSCSVMSEEP EGRSYSTLTT VREIETQTEL LSPGSGRAEE EEDQDEGIKQ AMNHFVQENG TLRAKPTGNG IYINGRGHLV |
| 45 | Vδ2 | | MQRISSLIHLSLFWAGVMSAIELVPEHQTVPVSI GVPATLRCSMKGEAIGNYYINWYRKTQGNTMTFI YREKDIYGPGFKDNFQGDIDIAKNLAVLKILAPSE RDEGSYYCACDTLGMGGEYTDKLIFGKGTRVTV EPRSQPHTKPSVFVMKNGTNVACLVKEFYPKDIR INLVSSKKITEFDPAIVISPSGKYNAVKLGKYEDS NSVTCSVQHDNKTVHSTDFEVKTDSTDHVKPKE TENTKQPSKSCHKPKAIVHTEKVNMMSLT |
| 46 | | Modified hinge | AAASDKTHTCPPCP |
| 47 | 5C8 | VHH | EVQLVESGGGLVQAGGSLRLSCAASGRPFSNYA MGWFRQAPGKEREFVAAISWSGGSTSYADSVK GRFTISRDNAKNTVYLQMNSPKPEDTAIYYCAAQ FSGADYGFGRLGIRGYEYDYWGQGTQVTVSS |
| 48 | LV1181-LFLE-Fc | VHH-Fc | EVQLVESGGGLVQAGGSLRLSCAASGSISSINLM GWHRQAPGKQRELVATITRGGSTNYADSVKGRF TISRDNGKNTVYLQMNSLKPEDTAAYYCVDVEPS GMGWRDYWGQGTQVTVSSAAASDKTHTCPPCP APEFEGGPSVFLFPPKPKDTLMISRTPEVTCVVVD VSHEDPEVKFNWYVDGVEVHNAKTKPREEQYNS TYRVVSVLTVLHQDWLNGKEYKCKVSNKALPAPI EKTISKAKGQPREPQVYTLPPSRDELTKNQVSLS CAVKGFYPSDIAVEWESNGQPENNYKTTPPVLDS DGSFFLVSKLTVDKSRWQQGNVFSCSVMHEALH NHYTQKSLSLSPGX4 |
| | | | wherein X₄ is a K that may or may not be present |
| 49 | LV1184-LFLE-Fc | VHH-Fc | EVQLVESGGGLVQAGGSLRLSCAASGSISSINLM GWFRQAPAKQRELVTSISPGGSVRYADSVKGRF |
| | | | TISRDVAKNTVDLQMNSLKPEDTAVYYCAAESER TYYFDSWGQGTQVTVSSAAASDKTHTCPPCPAP EFEGGPSVFLFPPKPKDTLMISRTPEVTCVVVDVS HEDPEVKFNWYVDGVEVHNAKTKPREEQYNSTY RVVSVLTVLHQDWLNGKEYKCKVSNKALPAPIEK TISKAKGQPREPQVYTLPPSRDELTKNQVSLSCA VKGFYPSDIAVEWESNGQPENNYKTTPPVLDSD GSFFLVSKLTVDKSRWQQGNVFSCSVMHEALHN HYTQKSLSLSPGX4 |
| | | | wherein X₄ is a K that may or may not be present |
| 50 | LV1185-LFLE-Fc | VHH-Fc | EVQLVESGGGLVQAGGSLRLSCAASRSTFSLNIM GWYRQAPGKQREYVATITTGGSTNYADSVRGRF TISRDNAEDTVYLQMNSLKPEDTAVYYCTAELVR RGPTTYWGRGTQVTVSSAAASDKTHTCPPCPAP EFEGGPSVFLFPPKPKDTLMISRTPEVTCVVVDVS HEDPEVKFNWYVDGVEVHNAKTKPREEQYNSTY RVVSVLTVLHQDWLNGKEYKCKVSNKALPAPIEK TISKAKGQPREPQVYTLPPSRDELTKNQVSLSCA VKGFYPSDIAVEWESNGQPENNYKTTPPVLDSD GSFFLVSKLTVDKSRWQQGNVFSCSVMHEALHN HYTQKSLSLSPGX4 |
| | | | wherein X₄ is a K that may or may not be present |
| 51 | LV1186-LFLE-Fc | VHH-Fc | EVQLVESGGGLVQAGGSLRLSCAASRSTFSLNIM GWYRQAPGKQREYVATITSGGSTNYADSVRGRF TISRDNAENTVYLQMDSLKPEDTAVYYCTAELVR RGPTTYWGRGTQVTVSSAAASDKTHTCPPCPAP EFEGGPSVFLFPPKPKDTLMISRTPEVTCVVVDVS |
| | | | HEDPEVKFNWYVDGVEVHNAKTKPREEQYNSTY RVVSVLTVLHQDWLNGKEYKCKVSNKALPAPIEK TISKAKGQPREPQVYTLPPSRDELTKNQVSLSCA VKGFYPSDIAVEWESNGQPENNYKTTPPVLDSD GSFFLVSKLTVDKSRWQQGNVFSCSVMHEALHN HYTQKSLSLSPGX4 |
| | | | wherein X₄ is a K that may or may not be present |
| 52 | 5C8var-Fc | VHH-Fc | EVQLLESGGGSVQPGGSLRLSCAASGRPFSNYA MSWFRQAPGKEREFVSAISWSGGSTSYADSVK GRFTISRDNSKNTLYLQMNSLRAEDTAVYYCAAQ FSGADFGFGALGIRGYEYDYWGQGTQVTVSSAA ASDKTHTCPPCPAPEFEGGPSVFLFPPKPKDTLMI SRTPEVTCVVVDVSHEDPEVKFNWYVDGVEVHN AKTKPREEQYNSTYRVVSVLTVLHQDWLNGKEY KCKVSNKALPAPIEKTISKAKGQPREPQVYTLPPS RDELTKNQVSLWCLVKGFYPSDIAVEWESNGQP ENNYKTTPPVLDSDGSFFLYSKLTVDKSRWQQG NVFSCSVMHEALHNHYTQKSLSLSPGX4 |
| | | | wherein X₄ is a K that may or may not be present |
| 53 | LV1181_H1 | VHH | EVQLVESGGGLVQPGGSLRLSCAASGSISSINLM GWHRQAPGKQRELVATITRGGSTNYADSVKGRF TISRDNGKNTVYLQMNSLRAEDTAAYYCVDVEPS GMGWRDYWGQGTQVTVSS |
| 54 | LV1181_H2 | VHH | EVQLVESGGGLVQPGGSLRLSCAASGSISSINLM GWHRQAPGKQRELVATITRGGSTNYADSVKGRF TISRDNGKNTLYLQMNSLRAEDTAAYYCVDVEPS GMGWRDYWGQGTQVTVSS |
| 55 | LV1181_H3 | VHH | EVQLVESGGGLVQPGGSLRLSCAASGSISSINLM GWHRQAPGKGLEWVATITRGGSTNYADSVKGR FTISRDNSKNTLYLQMNSLRAEDTAAYYCVDVEP SGMGWRDYWGQGTLVTVSS |
| 56 | LV1184_H1 | VHH | EVQLVESGGGLVQPGGSLRLSCAASGSISSINLM GWFRQAPAKQRELVTSISPGGSVRYADSVKGRF TISRDVAKNTVDLQMNSLRAEDTAVYYCAAESER TYYFDSWGQGTQVTVSS |
| 57 | LV1184_H2 | VHH | EVQLVESGGGLVQPGGSLRLSCAASGSISSINLM GWFRQAPAKQRELVTSISPGGSVRYADSVKGRF TISRDVSKNTVYLQMNSLRAEDTAVYYCAAESER TYYFDSWGQGTQVTVSS |
| 58 | LV1184_H3 | VHH | EVQLVESGGGLVQPGGSLRLSCAASGSISSINLM GWFRQAPAKGLEWVTSISPGGSVRYADSVKGRF TISRDNSKNTLYLQMNSLRAEDTAVYYCAAESER TYYFDSWGQGTQVTVSS |
| 59 | LV1185_H1 | VHH | EVQLVESGGGLVQPGGSLRLSCAASRSTFSLNIM GWYRQAPGKQREYVATITTGGSTNYADSVRGRF TISRDNAEDTVYLQMNSLRAEDTAVYYCTAELVR RGPTTYWGRGTQVTVSS |
| 60 | LV1185_H2 | VHH | EVQLVESGGGLVQPGGSLRLSCAASRSTFSLNIM GWYRQAPGKQREYVATITTGGSTNYADSVRGRF TISRDNSEDTVYLQMNSLRAEDTAVYYCTAELVR RGPTTYWGRGTQVTVSS |
| 61 | LV1185_H3 | VHH | EVQLVESGGGLVQPGGSLRLSCAASRSTFSLNIM GWYRQAPGKQREYVATITTGGSTNYADSVRGRF TISRDNSKNTLYLQMNSLRAEDTAVYYCTAELVR RGPTTYWGRGTLVTVSS |
| 62 | LV1186_H1 | VHH | EVQLVESGGGLVQPGGSLRLSCAASRSTFSLNIM GWYRQAPGKQREYVATITSGGSTNYADSVRGRF TISRDNAENTVYLQMDSLRAEDTAVYYCTAELVR RGPTTYWGRGTQVTVSS |
| 63 | LV1186_H2 | VHH | EVQLVESGGGLVQPGGSLRLSCAASRSTFSLNIM GWYRQAPGKQREYVATITSGGSTNYADSVRGRF TISRDNSENTVYLQMDSLRAEDTAVYYCTAELVR RGPTTYWGRGTQVTVSS |
| 64 | LV1186_H3 | VHH | EVQLVESGGGLVQPGGSLRLSCAASRSTFSLNIM GWYRQAPGKGLEWVATITSGGSTNYADSVRGR FTISRDNSKNTLYLQMDSLRAEDTAVYYCTAELVR RGPTTYWGRGTLVTVSS |
| 65 | LV1178 | VHH | EVQLVESGGALVQAGGSLRLSCSVSGLTASRLTM GWFRQAPGKEREFVARVYASGGLTDYADSVKGR FTISRDNAKNTVYLQMNSLEPEDTAVYYCVAGLW ADMRTMTSTRGYWGQGTQVTVSS |
| 66 | LV1178 | CDR1 | RLTMG |
| 67 | LV1178 | CDR2 | RVYASGGLTDYADSVKG |
| 68 | LV1178 | CDR3 | GLWADMRTMTSTRGY |
| 69 | LV1183 | VHH | EVQLVESGGGLVQAGGSLRLSCAASGSIFDINLM GWYRQAPGKLRELVASITRGGSTWYVDSVKGRF IISRDSAKNTVYLQMNSLKPGDTAVYYCAAESIG SYTFDRWGQGTQVTVSS |
| 70 | LV1183 | CDR1 | INLMG |
| 71 | LV1183 | CDR2 | SITRGGSTWYVDSVKG |
| 72 | LV1183 | CDR3 | ESIGSYTFDR |
| 73 | LV1187 | VHH | EVQLVESGGGLVQAGGFLRLSCAASRSVFRINV MGWYRQAPGKQRELVAGITSGGSRRLADSVKG |
| | | | RFTISRDNAKNTVYLQMNSLKPEDTAVYYCFAEA VVGDEPYWGQGTQVTVSS |
| 74 | LV1187 | CDR1 | INVMG |
| 75 | LV1187 | CDR2 | GITSGGSRRLADSVKG |
| 76 | LV1187 | CDR3 | EAVVGDEPY |

All references, articles, publications, patents, patent publications, and patent applications cited herein are incorporated by reference in their entireties for all purposes. However, mention of any reference, article, publication, patent, patent publication, and patent application herein is not, and should not be, taken as acknowledgment or any form of suggestion that they constitute valid prior art or form part of the common general knowledge in any country in the world.

### EXAMPLES

### Example 1: Generation of anti-Nectin-4 VHH

*Lama glama* (2 animals) were immunized with 4 injections of HepG2 cells (day 0, 14, 28 and 35) followed by 2 injections of human Nectin-4 protein (Sino Biologics) (day 49 and 63).

Serum was collected before, during and after immunization. Serially obtained pre-immune and immune sera were tested for the presence of anti-Nectin-4 antibodies by ELISA.

After confirmation of a Nectin-4 specific immune response, a whole blood sample was collected for peripheral blood lymphocyte (PBL) isolation. Phage libraries were constructed: total RNA was extracted from the peripheral blood lymphocytes, transcribed into cDNA, purified and used as a template for immunoglobulin heavy-chain-encoding cDNA amplification. Agarose gel electrophoresed purified genes encoding heavy-chain-only immunoglobulin (~ 700 bp) were excised from the gel, purified and used as a template for a nested PCR that introduced an appropriate 5' flanking restriction site and amplified only VHH-encoding cDNA. The purified PCR product was digested with appropriate restriction enzymes, after which the digested products were ligated in frame with geneIII into the pQ81 phagemid vector.

The ligated library was transformed into TG1 *E. coli* by electroporation for display on filamentous bacteriophage. Two consecutive rounds of selection were performed, followed by screening single clones for binding to the recombinant captured antigen. Those clones that scored positive for binding were sequenced and a selection of clones having a different sequence were then tested for Nectin-4 binding by ELISA. Nine different clones were identified and termed: LV1178, LV1179, LV1180, LV1181, LV1183, LV1184, LV1185, LV1186, LV1187.

### Example 2: Binding of VHH to human, cynomolgus monkey and mouse Nectin-4 expressing cells using cell-based ELISA

Protein sequences of human (Uniprot number Q96NY8), cynomolgus monkey (Uniprot number A0A2K5WES1) and mouse (Uniprot number Q8R007) Nectin-4 were reverse-translated to cDNA and then codon-optimized for expression in human cells. Regulatory elements were added to each cDNA: an N-terminal Kozak sequence and C-terminal stop codon (including *BamH*1 and *Age*1 restriction sites for cloning) and the cDNA was made as a synthetic gene. cDNAs were cloned into a suitable vector and their sequences were verified. Expression of the proteins was performed by transient transfection of the resulting plasmids in CHO-K1 cells. Expression of human/cynomolgus/mouse Nectin-4 was confirmed using commercially available control antibodies (Nectin-4 -AF647 [R&D systems] and Nectin-4 Antibody, anti-human, APC, REAfinity [Miltenyi]) in flow cytometry.

Cells from each transfection were seeded in 96F CellBIND plates (Corning) and cultured overnight at 37°C/5% CO₂ in a humidified atmosphere and subsequently incubated with serial dilutions of VHH, ranging from 0.032 to 500 nM for one hour at 37°C/5% CO₂. Plates were washed and cells were incubated with HRP-labeled rabbit anti-camelid VHH cocktail (GenScript) for one hour at 37°C/5% CO₂. After washing, 3,3',5,5'-tetramethylbenzidine (TMB) substrate was added, followed by 20 minute incubation at room temperature. The reaction was stopped by addition of Stop buffer (1M H₂SO₄) and the optical densities were measured with a multimode plate reader Spectramax iD5, at wavelengths 450 nm and 610 nm.

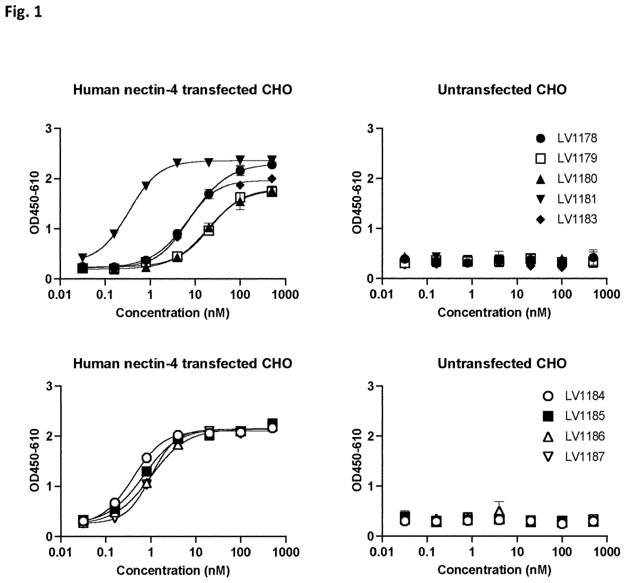

Figure 1 shows that all VHH bound dose-dependently to human Nectin-4 expressing CHO-K1 cells, and not to non-transfected CHO-K1 cells. Nectin-4 binding VHHs could be divided in three groups; strong binders LV1181, LV1184, LV1185, LV1186 and LV1187, intermediate binders LV1178, LV1183 and weak binders LV1179 and LV1180.

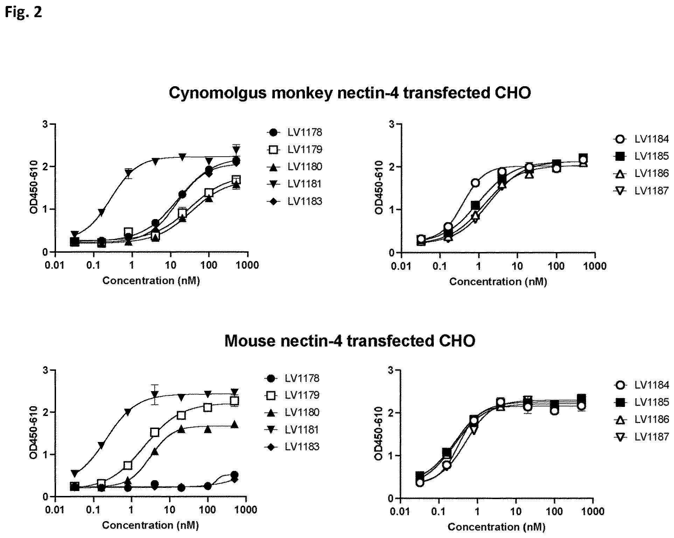

All VHH showed cross reactivity to CHO-K1 cells expressing cynomolgus monkey Nectin-4 (Figure 2). Again, the three groups of strong, intermediate and weak binding VHHs were observed. All 'stronger binding' VHHs also cross-reacted to murine Nectin-4, as did the 'weaker binding' VHH. The intermediate binding VHHs LV1178 and LV1183 did not bind to CHO-K1 cells expressing mouse Nectin-4.

### Example 3: Binding of VHHs to tumor cell lines

Binding of the selected VHHs to Nectin-4 endogenously expressed on human tumor cell lines was determined by flow cytometry. Nectin-4 expression on tumor cell lines BeWo (Sigma), A-431 (ATCC) and HT-29 (ATCC) was confirmed using commercially available monoclonal antibodies (Nectin-4-AF647 [R&D systems] and Nectin-4 Antibody, anti-human, APC, REAfinity [Miltenyi]). Cell line U251 was used as Nectin-4 negative cell line.

Cells were incubated with a concentration range of each VHH, ranging from 0.032 to 100 nM and VHH binding was detected using a rabbit anti-camelid iFluor 647-labeled antibody (Genscript). Staining was visualized using a FACS Celesta (Becton and Dickinson).

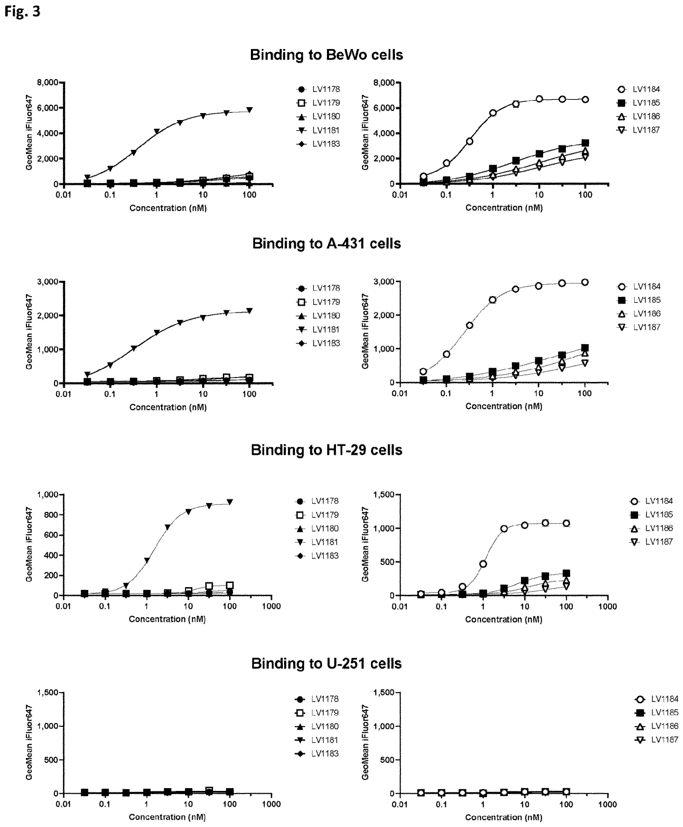

Figure 3 shows that LV1181 and LV1184 bound readily to Nectin-4 expressing tumor cells. LV1185, LV1186 and LV1187 showed less, yet still detectable binding, whereas LV1178, LV1179 and LV1180 showed very limited binding to Nectin-4 expressing tumor cells. EC50s for binding to the BeWo, A431 and HT-29 cells for LV1181 and LV1184 were determined by curve fitting using GraphPad-Prism software and are shown in Table 2; EC50s for binding of the other VHHs could not be determined due to incomplete binding curves.

**Table 2: EC50 values for VHH binding to tumor cells**

| **Tumor cell line** | **EC50 LV1181 (nM)** | **EC50 LV1184 (nM)** |
|---|---|---|
| BeWo | 0.4 | 0.3 |
| A431 | 0.33 | 0.24 |
| HT-29 | 1.5 | 1.2 |

### Example 4: Synthetic gene synthesis, production and purification of bispecific VHH

The sequences of the four Nectin-4-specific VHH domain antibodies that showed strongest binding to tumor cells that endogenously express Nectin-4, i.e. LV1181, LV1184, LV1185 and LV1186 were then re-formatted to bispecific VHHs with a Vδ2 specific VHH (5C8 (SEQ ID NO:47) (SEQ ID NO:59 of WO2015156673) in the orientation: N-term-anti-Nectin-4 VHH-linker-anti-Vδ2 VHH. The linker between the two VHH 'heads' was a glycine(G)-serine(S) stretch with the sequence G4S. The cDNAs encoding these proteins were made by synthetic gene assembly and cloned into a proprietary expression vector. The proteins were expressed by transient transfection of the encoding plasmids to HEK293E-253 cells and then (after 6 days of expression) purified from the conditioned cell culture supernatant using HiTrap Fibro PrismA columns according to the supplier's protocol. After elution, proteins were buffer-exchanged to PBS. Purified bispecific VHH was always >95% pure as determined by SDS-PAGE analysis using Coomassie staining and contained very low levels of endotoxin (<0.5 EU/mg).

### Example 5: Affinity determination of VHHs and bispecific VHHs for binding to human Nectin-4 using biolayer interferometry (BLI)

To determine the kinetics of binding of the nine VHHs and four Nectin-4xVδ2 bispecific VHHs derived thereof to human Nectin-4, recombinant purified human Nectin-4-Fc fusion protein (Aero Biosystems) was loaded onto anti-human IgG Fc Capture biosensors (using a concentration of 2.5 µg/mL) for an Octet Red96e (Sartorius) instrument. The sensors were then dipped in different concentrations of VHHs; dilutions were made in 10x kinetic buffer (10xKB) provided by the supplier. From the obtained sensorgrams, the kinetic association- and dissociation rate constants were determined by curve fitting.

Table 3 shows the binding affinities (K_{D}) of the VHHs determined in 2 independent runs.

**Table 3: Binding affinities (K_{D}) of the VHHs for human Nectin-4 determined in 2 independent BLI runs.**

| **VHH** | **KD (nM) Run 1** | **KD (nM) Run 2** |
|---|---|---|
| LV1178 | ND | 8.02 |
| LV1179 | 12.86 | 7.33 |
| LV1180 | 10.43 | 7.8 |
| LV1181 | 0.8 | 0.76 |
| LV1183 | 5.7 | 7.98 |
| LV1184 | 0.51 | 0.51 |
| LV1185 | 3.61 | 2.34 |
| LV1186 | 3.93 | 3.94 |
| LV1187 | 5.2 | 6.09 |

Table 4 shows the kinetic parameters of binding of the Nectin-4xVδ2 bispecific VHHs for human Nectin-4 determined in 2 independent runs. ND means Not Determined.

**Table 4: Binding kinetics of Nectin-4xVδ2 bispecific VHHs for human Nectin-4 determined in 2 independent BLI runs.**

| **Bispecific VHH** | **Run number** | **K_{D} (nM)** | **Kₒₙ (1/Ms)** | **K_{dis} (1/s)** |
|---|---|---|---|---|
| LV1181xVδ2 | 1 | 0.26 | 1.04E+06 | 2.66E-04 |
| | 2 | 0.37 | 8.18E+05 | 3.00E-04 |
| LV1184xVδ2 | 1 | 0.07 | 9.73E+05 | 6.33E-05 |
| | 2 | 0.18 | 8.44E+05 | 1.49E-04 |
| LV1185xVδ2 | 1 | 2.0 | 6.42E+05 | 1.27E-03 |
| | 2 | 2.4 | 5.93E+05 | 1.44E-03 |
| LV1186xVδ2 | 1 | 2.3 | 7.31E+05 | 1.65E-03 |
| | 2 | 2.6 | 6.60E+05 | 1.70E-03 |

### Example 6: Competition for binding to human Nectin-4

Using BLI, competition for binding to human Nectin-4 between the VHHs was determined. Anti-human IgG Fc Capture sensors were loaded with human Nectin-4 Fc fusion protein as described above. The nine different VHHs identified were used in concentrations based on the affinities as indicated above. A fixed amount of the first VHH was allowed to bind to the Nectin-4 loaded sensor, after which the sensor was exposed to a mixture of the same amount of first VHH and a fixed amount of second VHH, followed by dissociation in 10X KB.

From the obtained sensorgrams it was concluded that all nine different VHHs competed with each other for binding to human Nectin-4.

### Example 7: Binding of Nectin-4xVδ2 bispecific VHHs to tumor cell lines

Binding of the Nectin-4xVδ2 bispecific VHHs to Nectin-4 endogenously expressed on human tumor cell lines A-431 and HT-29 was determined by flow cytometry as described above.

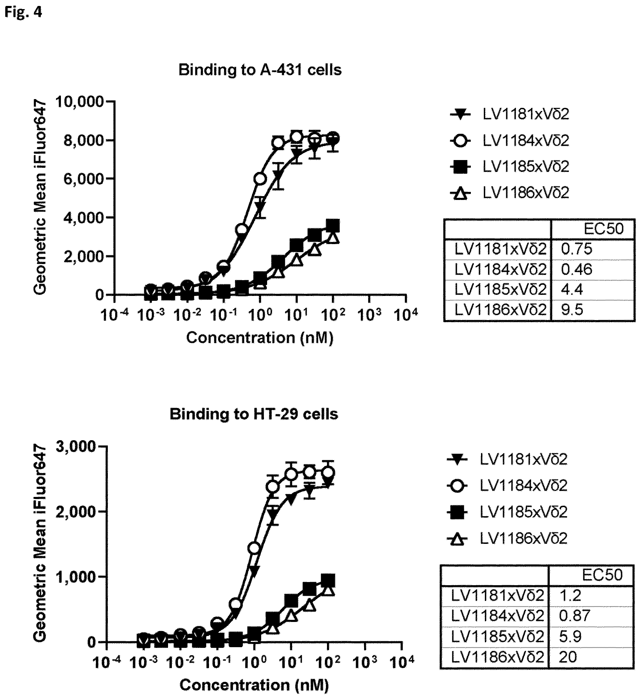

Figure 4 shows that LV1181xVδ2 and LV1184xVδ2 bound to both cell lines with high affinity (EC50s ranging from 0.46 to 1.2 nM). Both LV1185xVδ2 as well as LV1186xVδ2 bound to the tumor cell lines, yet with lower affinity.

### Example 8: PBMC isolation and generation of human donor-derived Vγ9Vδ2-T cell cultures

Buffy coats were obtained from blood supply service Sanquin and used for isolation of peripheral blood mononuclear cells (PBMC). PBMC were isolated using Lymphoprep^{™} density gradient centrifugation. Vγ9Vδ2-T cells were then isolated from healthy donor-derived PBMC by magnetic-activated cell sorting (MACS) using a FITC-labeled anti-TCR Vδ2 mouse monoclonal antibody (Mab) in combination with goat anti-mouse IgG microbeads. Purified Vγ9Vδ2-T cells were stimulated every seven days with a feeder cell mix consisting of irradiated PBMC from two (other) healthy donors and an Epstein Barr Virus transformed B cell line (JY) resuspended in Roswell Park Memorial Institute (RPMI) medium supplemented with 10 IU/mL IL-7, 10 ng/mL IL-15 and 50 ng/ml PHA. Expanded Vγ9Vδ2-T cell cultures were tested for purity (and only used when >90% pure) before being used for experiments.

### Example 9: Binding of Nectin-4xVδ2 bispecific VHHs to Vγ9Vδ2 T cells

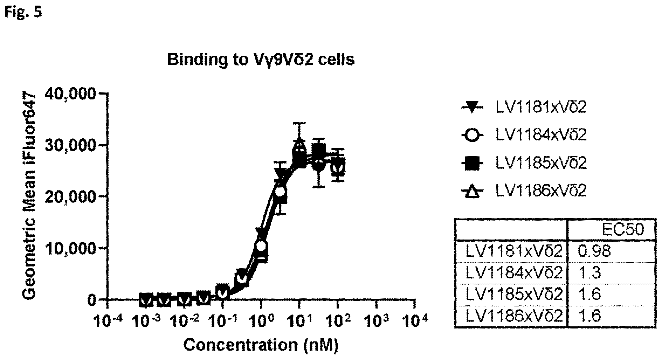

Binding of the Nectin-4xVδ2 bispecific VHHs to **Vγ9Vδ2** T cells was determined by flow cytometry essentially as described above. Figure 5 shows that all 4 Nectin-4xVδ2 bispecific VHHs bound to **Vγ9Vδ2** T cells with similar EC50 of 1-1.6 nM.

### Example 10: Vγ9Vδ2-T cell degranulation and cytotoxicity of A-431 cells induced by Nectin-4xVδ2 bispecific VHHs

For a Vγ9Vδ2-T cell activation and cytotoxicity assay, A-431 tumor target cells and **Vγ9Vδ2** T cells were mixed in a 1:1 ratio in culture medium and labeled anti-CD107A antibody was added. This mixture was seeded in 96-well culture plates (with 50,000 target/Vγ9Vδ2 T cells per well) and serial dilutions of Nectin-4xVδ2 bispecific VHHs were added. Controls (tumor cells and Vγ9Vδ2-T cells alone with and without the highest concentration of Nectin-4xVδ2 bispecific VHH) were also prepared. After incubation for 24 hours at 37°C/5% CO₂, cells were resuspended, washed and transferred to 96-well U bottom FACS plates. Remaining adherent cells were detached using trypsin/0.5% EDTA, washed and transferred to the corresponding wells of the FACS plates. Plates were washed, cells were resuspended in FACS buffer (PBS/1% BSA) and incubated with labeled anti-CD3 (Biolegend) and anti-Vγ9 (Beckman Coulter) antibodies for 30 minutes at 4°C. Cells were washed and labeled 7-AAD was added. Staining was visualized using a FACS Celesta (Becton and Dickinson).

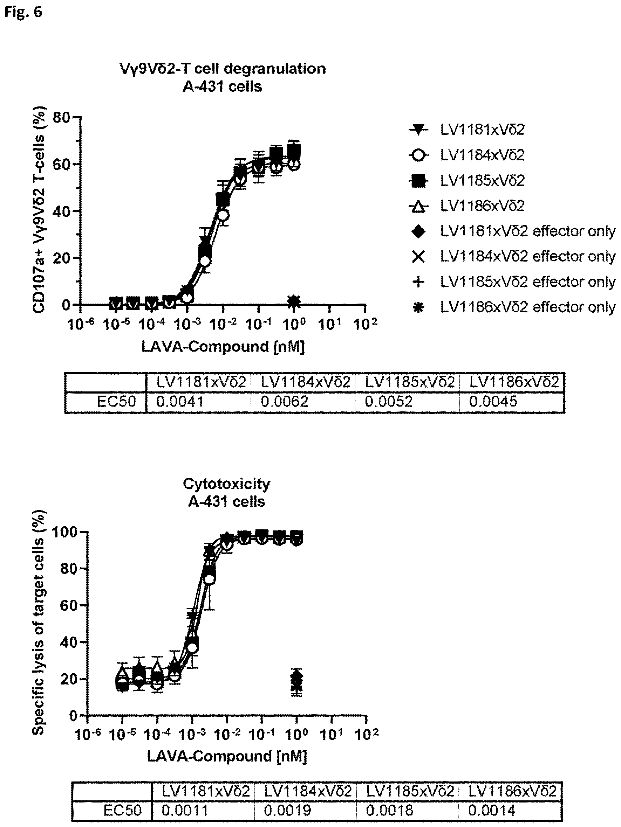

Figure 6 shows that all four Nectin-4xVδ2 bispecific VHHs induced similar levels of **Vγ9Vδ2** T cell degranulation as determined by CD107a expression on the T cells. Surprisingly, despite differences in binding to tumor cells as described above, all four Nectin-4xVδ2 bispecific VHHs induced the same level of lysis of A431 cells with similar EC50s.

### Example 11: Humanization of the anti-Nectin-4 VHHs

The amino acid sequence of the llama-derived anti-Nectin-4 VHHs (LV1181, LV1184, LV1185 and LV1186) were aligned to the human V gene database and the closest human germline match was found to be IGHV3-66*01. Based on sequence differences in the framework regions between the human and llama-derived sequence, for each anti-Nectin-4 VHH three humanized variants (SEQ ID NO:53-64) were designed.

## Claims

1. A multispecific antibody comprising a first antigen-binding region capable of binding human Nectin-4 and a second antigen-binding region capable of binding a human Vγ9Vδ2 T cell receptor, wherein the multispecific antibody is capable of competing for binding to human Nectin-4 with an antibody having the sequence set forth in SEQ ID NO: 1.

2. The multispecific antibody according to claim 1, wherein the multispecific antibody is a bispecific antibody.

3. The multispecific antibody according to any one of the preceding claims, wherein the first antigen-binding region is a single-domain antibody.

4. The multispecific antibody according to any one of the preceding claims, wherein the multispecific antibody is capable of competing for binding to human Nectin-4 with: an antibody having the sequence set forth in SEQ ID NO:9 or an antibody having the sequence set forth in SEQ ID NO:17 or an antibody having the sequence set forth in SEQ ID NO:25, preferably wherein the multispecific antibody is capable of competing for binding to human Nectin-4 with: an antibody having the sequence set forth in SEQ ID NO:9, an antibody having the sequence set forth in SEQ ID NO: 17 and an antibody having the sequence set forth in SEQ ID NO:25.

5. The multispecific antibody according to any one of the preceding claims, wherein the first antigen-binding region comprises:
• the CDR1 sequence set forth in SEQ ID NO:2, the CDR2 sequence set forth in SEQ ID NO:3 and the CDR3 sequence set forth in SEQ ID NO:4, or
• a CDR1 sequence which differs from the CDR1 sequence set forth in SEQ ID NO:2 in one or two positions, a CDR2 sequence which differs from the CDR2 sequence set forth in SEQ ID NO:3 in one, two, three, four or five positions and a CDR3 sequence which differs from the CDR3 sequence set forth in SEQ ID NO:4 in one or two positions,
wherein preferably the first antigen-binding region comprises or consists of:
• the sequence set forth in SEQ ID NO: 1, SEQ ID NO:56, SEQ ID NO:57 or SEQ ID NO:58, or
• a sequence having at least 90%, such as least 92%, e.g. at least 94%, such as at least 96%, e.g. at least 98% sequence identity to the sequence ser forth in SEQ ID NO: 1.

6. The multispecific antibody according to any one of claims 1 to 4, wherein the first antigen-binding region comprises:
• the CDR1 sequence set forth in SEQ ID NO:10, the CDR2 sequence set forth in SEQ ID NO:11 and the CDR3 sequence set forth in SEQ ID NO:12, or
• a CDR1 sequence which differs from the CDR1 sequence set forth in SEQ ID NO:10 in one or two positions, a CDR2 sequence which differs from the CDR2 sequence set forth in SEQ ID NO:11 in one, two, three, four or five positions and a CDR3 sequence which differs from the CDR3 sequence set forth in SEQ ID NO:12 in one or two positions,
wherein preferably the first antigen-binding region comprises or consists of:
• the sequence set forth in SEQ ID NO:9, SEQ ID NO:53, SEQ ID NO:54 or SEQ ID NO:55, or
• a sequence having at least 90%, such as least 92%, e.g. at least 94%, such as at least 96%, e.g. at least 98% sequence identity to the sequence ser forth in SEQ ID NO:9.

7. The multispecific antibody according to any one of the claims 1 to 4, wherein the first antigen-binding region comprises:
• the CDR1 sequence set forth in SEQ ID NO:18, the CDR2 sequence set forth in SEQ ID NO:19 and the CDR3 sequence set forth in SEQ ID NO:20, or
• a CDR1 sequence which differs from the CDR1 sequence set forth in SEQ ID NO:18 in one or two positions, a CDR2 sequence which differs from the CDR2 sequence set forth in SEQ ID NO:19 in one, two, three, four or five positions and a CDR3 sequence which differs from the CDR3 sequence set forth in SEQ ID NO:20 in one or two positions,
wherein preferably the first antigen-binding region comprises or consists of:
• the sequence set forth in SEQ ID NO: 17, SEQ ID NO:59, SEQ ID NO:60 or SEQ ID NO:61, or
• a sequence having at least 90%, such as least 92%, e.g. at least 94%, such as at least 96%, e.g. at least 98% sequence identity to the sequence ser forth in SEQ ID NO: 17.

8. The multispecific antibody according to any one of claims 1 to 4, wherein the first antigen-binding region comprises:
• the CDR1 sequence set forth in SEQ ID NO:26, the CDR2 sequence set forth in SEQ ID NO:27 and the CDR3 sequence set forth in SEQ ID NO:28, or
• a CDR1 sequence which differs from the CDR1 sequence set forth in SEQ ID NO:26 in one or two positions, a CDR2 sequence which differs from the CDR2 sequence set forth in SEQ ID NO:27 in one, two, three, four or five positions and a CDR3 sequence which differs from the CDR3 sequence set forth in SEQ ID NO:28 in one or two positions,
wherein preferably the first antigen-binding region comprises or consists of:
• the sequence set forth in SEQ ID NO:25, SEQ ID NO:62, SEQ ID NO:63 or SEQ ID NO:64, or
• a sequence having at least 90%, such as least 92%, e.g. at least 94%, such as at least 96%, e.g. at least 98% sequence identity to the sequence ser forth in SEQ ID NO:25.

9. The multispecific antibody according to any one of claims 1 to 4, wherein the first antigen-binding region comprises:
• the CDR1 sequence set forth in SEQ ID NO:66, the CDR2 sequence set forth in SEQ ID NO:67 and the CDR3 sequence set forth in SEQ ID NO:68, or
• a CDR1 sequence which differs from the CDR1 sequence set forth in SEQ ID NO:66 in one or two positions, a CDR2 sequence which differs from the CDR2 sequence set forth in SEQ ID NO:67 in one, two, three, four or five positions and a CDR3 sequence which differs from the CDR3 sequence set forth in SEQ ID NO:68 in one or two positions,
wherein preferably the first antigen-binding region comprises or consists of:
• the sequence set forth in SEQ ID NO:65, or
• a sequence having at least 90%, such as least 92%, e.g. at least 94%, such as at least 96%, e.g. at least 98% sequence identity to the sequence ser forth in SEQ ID NO:65.
or wherein the first antigen-binding region comprises:
• the CDR1 sequence set forth in SEQ ID NO:70, the CDR2 sequence set forth in SEQ ID NO:71 and the CDR3 sequence set forth in SEQ ID NO:72, or
• a CDR1 sequence which differs from the CDR1 sequence set forth in SEQ ID NO:70 in one or two positions, a CDR2 sequence which differs from the CDR2 sequence set forth in SEQ ID NO:71 in one, two, three, four or five positions and a CDR3 sequence which differs from the CDR3 sequence set forth in SEQ ID NO:72 in one or two positions,
wherein preferably the first antigen-binding region comprises or consists of:
• the sequence set forth in SEQ ID NO:69, or
• a sequence having at least 90%, such as least 92%, e.g. at least 94%, such as at least 96%, e.g. at least 98% sequence identity to the sequence ser forth in SEQ ID NO:69.
or wherein the first antigen-binding region comprises:
• the CDR1 sequence set forth in SEQ ID NO:74, the CDR2 sequence set forth in SEQ ID NO:75 and the CDR3 sequence set forth in SEQ ID NO:76, or
• a CDR1 sequence which differs from the CDR1 sequence set forth in SEQ ID NO:74 in one or two positions, a CDR2 sequence which differs from the CDR2 sequence set forth in SEQ ID NO:75 in one, two, three, four or five positions and a CDR3 sequence which differs from the CDR3 sequence set forth in SEQ ID NO:76 in one or two positions,
wherein preferably the first antigen-binding region comprises or consists of:
• the sequence set forth in SEQ ID NO:73, or
• a sequence having at least 90%, such as least 92%, e.g. at least 94%, such as at least 96%, e.g. at least 98% sequence identity to the sequence ser forth in SEQ ID NO:73.

10. The multispecific antibody according to any one of the preceding claims, wherein the second antigen-binding region is a single-domain antibody.

11. The multispecific antibody according to any one of the preceding claims,
wherein the multispecific antibody is able to activate human Vγ9Vδ2 T cells, preferably wherein the second antigen-binding region is capable of binding the Vδ2 chain of a human Vγ9Vδ2 T cell receptor,
wherein preferably:
the multispecific antibody is capable of competing for binding to human Vδ2 with an antibody having the sequence set forth in SEQ ID NO:37, and/or
the multispecific antibody is capable of competing for binding to human Vδ2 with an antibody having the sequence set forth in SEQ ID NO:38.

12. The multispecific antibody according to any one of the preceding claims,
wherein the second antigen-binding region comprises the VH CDR1 sequence set forth in SEQ ID NO:34, the VH CDR2 sequence set forth in SEQ ID NO:35 and the VH CDR3 sequence set forth in SEQ ID NO:36, wherein preferably X₁ is S, X₂ is F and X₃ is A, and wherein most preferably the second antigen-binding region comprises or consists of the sequence set forth in SEQ ID NO:37, or a sequence having at least 90%, such as least 92%, e.g. at least 94%, such as at least 96%, e.g. at least 98% sequence identity to the sequence set forth in SEQ ID NO:37,
or
wherein the second antigen-binding region comprises the VH CDR1 sequence set forth in SEQ ID NO:39, the VH CDR2 sequence set forth in SEQ ID NO:40 and the VH CDR3 sequence set forth in SEQ ID NO:41, wherein preferably the second antigen-binding region comprises or consists of the sequence set forth in SEQ ID NO:38, or a sequence having at least 90%, such as least 92%, e.g. at least 94%, such as at least 96%, e.g. at least 98% sequence identity to the sequence set forth in SEQ ID NO:38.

13. The multispecific antibody according to any one of the preceding claims, wherein the multispecific antibody further comprises a half-life extension domain, such as an Fc region, preferably a human Fc region, wherein preferably the Fc region is a heterodimer comprising two Fc polypeptides, wherein the first antigen-binding region is fused to the first Fc polypeptide and the second antigen-binding region is fused to the second Fc polypeptide and wherein the first and second Fc polypeptides comprise asymmetric amino acid mutations that favor the formation of heterodimers over the formation of homodimers, wherein preferably the first Fc polypeptide comprises a T366W substitution and the second Fc polypeptide comprises T366S, L368A and Y407V substitutions, or vice versa, wherein the amino acid positions correspond to human IgG1 according to the EU numbering system.

14. The multispecific antibody according to claim 13, wherein the cysteine residues at position 220 in the first and second Fc polypeptides have been deleted or substituted, and wherein the first and second Fc polypeptides further comprise a mutation at position 234 and/or 235, preferably wherein the first and second Fc polypeptide comprise an L234F and an L235E substitution, wherein the amino acid positions correspond to human IgG1 according to the EU numbering system, wherein preferably the first Fc polypeptide comprises the sequence set forth in SEQ ID NO:42 and the second Fc polypeptide comprises the sequence set forth in SEQ ID NO:43, or vice versa.

15. An antibody comprising a first antigen-binding region capable of binding human Nectin-4, wherein the antibody is capable of competing for binding to human Nectin-4 with an antibody having the sequence set forth in SEQ ID NO:1, wherein preferably the first antigen-binding region is a single-domain antibody comprising:
(i) the CDR1 sequence set forth in SEQ ID NO:2, the CDR2 sequence set forth in SEQ ID NO:3 and the CDR3 sequence set forth in SEQ ID NO:4, or a CDR1 sequence which differs from the CDR1 sequence set forth in SEQ ID NO:2 in one or two positions, a CDR2 sequence which differs from the CDR2 sequence set forth in SEQ ID NO:3 in one, two, three, four or five positions and a CDR3 sequence which differs from the CDR3 sequence set forth in SEQ ID NO:4 in one or two positions,
(ii) the CDR1 sequence set forth in SEQ ID NO:10, the CDR2 sequence set forth in SEQ ID NO:11 and the CDR3 sequence set forth in SEQ ID NO:12, or a CDR1 sequence which differs from the CDR1 sequence set forth in SEQ ID NO:10 in one or two positions, a CDR2 sequence which differs from the CDR2 sequence set forth in SEQ ID NO:11 in one, two, three, four or five positions and a CDR3 sequence which differs from the CDR3 sequence set forth in SEQ ID NO:12 in one or two positions,
(iii) the CDR1 sequence set forth in SEQ ID NO:18, the CDR2 sequence set forth in SEQ ID NO:19 and the CDR3 sequence set forth in SEQ ID NO:20, or a CDR1 sequence which differs from the CDR1 sequence set forth in SEQ ID NO:18 in one or two positions, a CDR2 sequence which differs from the CDR2 sequence set forth in SEQ ID NO:19 in one, two, three, four or five positions and a CDR3 sequence which differs from the CDR3 sequence set forth in SEQ ID NO:20 in one or two positions, or
(iv) the CDR1 sequence set forth in SEQ ID NO:26, the CDR2 sequence set forth in SEQ ID NO:27 and the CDR3 sequence set forth in SEQ ID NO:28, or a CDR1 sequence which differs from the CDR1 sequence set forth in SEQ ID NO:26 in one or two positions, a CDR2 sequence which differs from the CDR2 sequence set forth in SEQ ID NO:27 in one, two, three, four or five positions and a CDR3 sequence which differs from the CDR3 sequence set forth in SEQ ID NO:28 in one or two positions, or
(v) the CDR1 sequence set forth in SEQ ID NO:66, the CDR2 sequence set forth in SEQ ID NO:67 and the CDR3 sequence set forth in SEQ ID NO:68, or a CDR1 sequence which differs from the CDR1 sequence set forth in SEQ ID NO:66 in one or two positions, a CDR2 sequence which differs from the CDR2 sequence set forth in SEQ ID NO:67 in one, two, three, four or five positions and a CDR3 sequence which differs from the CDR3 sequence set forth in SEQ ID NO:68 in one or two positions, or
(vi) the CDR1 sequence set forth in SEQ ID NO:70, the CDR2 sequence set forth in SEQ ID NO:71 and the CDR3 sequence set forth in SEQ ID NO:72, or a CDR1 sequence which differs from the CDR1 sequence set forth in SEQ ID NO:70 in one or two positions, a CDR2 sequence which differs from the CDR2 sequence set forth in SEQ ID NO:71 in one, two, three, four or five positions and a CDR3 sequence which differs from the CDR3 sequence set forth in SEQ ID NO:72 in one or two positions, or
(vii) the CDR1 sequence set forth in SEQ ID NO:74, the CDR2 sequence set forth in SEQ ID NO:75 and the CDR3 sequence set forth in SEQ ID NO:76, or a CDR1 sequence which differs from the CDR1 sequence set forth in SEQ ID NO:74 in one or two positions, a CDR2 sequence which differs from the CDR2 sequence set forth in SEQ ID NO:75 in one, two, three, four or five positions and a CDR3 sequence which differs from the CDR3 sequence set forth in SEQ ID NO:76 in one or two positions.

16. A pharmaceutical composition comprising a multispecific antibody according to any one of the claims 1 to 14 or the antibody according to claim 15 and a pharmaceutically-acceptable excipient.

17. The multispecific antibody according to any one of claims 1 to 14 or the antibody according to claim 15 for use as a medicament, preferably for use in the treatment of cancer, more preferably for use in the treatment of bladder cancer, breast cancer, renal cancer, prostate cancer, ovarian cancer, esophageal cancer, head and neck cancer, lung cancer, pancreatic cancer, gastric cancer, thyroid cancer, colorectal cancer, cholangiocarcinoma or uterine corpus endometrial carcinoma.

18. A nucleic acid construct comprising a nucleotide sequence encoding a multispecific antibody according to any one of claims 1 to 14 or the antibody according to claim 15 or a host cell comprising one or more nucleic acid constructs encoding a multispecific antibody according to any one of claims 1 to 14 or the antibody according to claim 15.
